(19) 
**Europäisches Patentamt**
**European Patent Office**
**Office européen des brevets**

(11) **EP 4 563 597 A1**

(12) **EUROPEAN PATENT APPLICATION**
published in accordance with Art. 153(4) EPC

(43) Date of publication:
04.06.2025 Bulletin 2025/23

(21) Application number: 23847000.9

(22) Date of filing: 26.07.2023

(51) International Patent Classification (IPC):
*C07K 16/28* (2006.01)        *A61K 47/68* (2017.01)
*A61P 35/00* (2006.01)        *A61K 39/00* (2006.01)

(52) Cooperative Patent Classification (CPC):
A61K 39/00; A61K 47/68; A61P 35/00; C07K 16/28

(86) International application number:
PCT/KR2023/010847

(87) International publication number:
WO 2024/025343 (01.02.2024 Gazette 2024/05)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: 26.07.2022 KR 20220092464

(71) Applicant: **AIMED BIO INC.**
**Seoul, 05835 (KR)**

(72) Inventors:
• **MIN, Byeongkwi**
**Seoul 05835 (KR)**
• **YOO, Minyoung**
**Seoul 05835 (KR)**
• **KIM, Jiwon**
**Seoul 05835 (KR)**
• **KIM, Jongbin**
**Seoul 05835 (KR)**

(74) Representative: **Mewburn Ellis LLP**
**Aurora Building**
**Counterslip**
**Bristol BS1 6BX (GB)**

Remarks:
The complete document including Reference
Table(s) and the Sequence Listing(s) can be
downloaded from the EPO website

(54) **ANTI-ROR1 ANTIBODY AND USE THEREOF**

(57) The present invention relates to: a receptor tyrosine kinase like orphan receptor 1 (ROR 1) antibody or an antigen-binding fragment thereof; a nucleic acid encoding same; a recombinant expression vector carrying the nucleic acid; a host cell transinfected with the recombinant expression vector; a method for preparing the antibody or the antigen-binding fragment thereof; a bi- or multi-specific antibody bearing the antibody or the antigen-binding fragment thereof; an immune cell-engaging bi- or multi-specific antibody; an antibody-drug conjugate (ADC) in which the antibody or the antigen-binding fragment thereof is bound to a drug; a chimeric antigen receptor (CAR) containing the scFv of the antibody as an antigen-binding site of an extracellular domain; an immune cell having the chimeric antigen receptor introduced thereinto; a composition for combination therapy including the antibody or the antigen-binding fragment thereof; a composition for preventing or treating cancer; and a method for preventing or treating cancer.

**EP 4 563 597 A1**

FIG. 1

**Description**

**TECHNICAL FIELD**

The present invention relates to an anti-ROR1 (receptor tyrosine kinase like orphan receptor 1) antibody or antigen-binding fragment thereof, a nucleic acid encoding the same, a recombinant expression vector comprising the nucleic acid, a host cell transfected with the recombinant expression vector, a method of producing the antibody or antigen-binding fragment thereof, a bispecific or multispecific antibody comprising the antibody or antigen-binding fragment thereof, an immune cell-engaging bispecific or multispecific antibody comprising an scFv composed of an scFv of the antibody and a second binding domain comprising at least one scFv of an antibody that binds to an immune cell-activating antigen, an antibody-drug conjugate (ADC) in which the antibody or antigen-binding fragment thereof is conjugated to a drug, a chimeric antigen receptor (CAR) comprising an scFv of the antibody as an antigen-binding site of an extracellular domain, an immune cell into which the chimeric antigen receptor is introduced, a composition for combination therapy, a composition for preventing or treating cancer, and a method of preventing or treating cancer, comprising the antibody or antigen-binding fragment thereof or the immune cell.

**BACKGROUND ART**

ROR (receptor tyrosine kinase like orphan receptor) is a representative group of transmembrane RTK (receptor tyrosine kinase) receptors that have diverse effects on the overall cell activity. The ROR family is composed of ROR1 and ROR2, and the amino acid sequence similarity between the two proteins amounts to approximately 60%. The ROR family includes Ig, cysteine-rich and kringle domains extracellularly, and tyrosine kinase, Ser/Thr-rich and proline-rich domains intracellularly.

ROR1 is expressed during embryogenesis and affects various early cell activities, but the expression thereof gradually decreases as adult tissue formation progresses. In addition, ROR1 is expressed in the intermediate stage of the normal B cell maturation process but is not expressed in mature B and T cells or monocytes. However, ROR1 is known to be overexpressed in various tumor cells and is thus considered to be a fetal gene for malignant tumors. It has been reported that ROR1 acts as a receptor for Wnt5a, activating non-canonical Wnt signals, resulting in cancer cell proliferation and metastasis. Accordingly, ROR1 has begun to emerge as a representative target for anticancer antibody therapy. Specifically, as it was discovered that ROR1 is overexpressed in chronic lymphocytic leukemia (CLL), thorough research has been conducted to determine whether ROR1 is overexpressed in various carcinomas (Klein et al., 2001, J. Exp. Med 194;1625). Thereby, ROR1 overexpression has been confirmed not only in a variety of blood cancers (acute myeloid leukemia (AML), acute lymphocytic leukemia (ALL), and diffuse large B-cell lymphoma (DLBCL)) in addition to chronic lymphocytic leukemia (CLL), but also in solid tumors such as breast cancer, cervical cancer, ovarian cancer, lung cancer, non-small cell lung cancer (NSCLC), and brain cancer.

Although ROR1 was initially classified as a pseudokinase and underestimated, it is known that overexpression of ROR1 in the cancer types described above is closely related to survival of cancer patients and cancer metastasis, and there is a need to develop effective therapeutic agents therefor. International Patent No. WO 2016/172726 A1 discloses an anti-ROR1 monoclonal antibody and the use thereof. This antibody shows ROR1-specific characteristics and binds to and kills CLL cells in which ROR1 is overexpressed, suggesting the potential for developing an antibody-based therapeutic agent that selectively recognizes ROR1.

Since anti-ROR1 antibodies have different characteristics, it is possible to develop various anticancer antibodies depending on the type of cancer in which the antigen is expressed. By targeting cancer types with a high recurrence rate, such as CLL, the unmet demand for anticancer antibodies may be satisfied (Choi et al., 2018, Cell Stem Cell 22, 951-959). Cancer-specific expression of ROR1 appears not only in cancer cells, but also in cancer stem cells or stemness, which helps suppress tumor metastasis or recurrence. Taking into consideration the expression thereof in various cancer-related cells, there are countless possibilities for developing various therapeutic agents based on existing antibodies.

Under this technical background, the present inventors have not only discovered antibodies specific to ROR1 but also selected antibodies capable of more selectively recognizing ROR1 on the surface of patient-derived cells and identified antibodies having superior anticancer efficacy and target accuracy compared to conventionally known therapeutic agents under development, thus culminating in the present invention.

**SUMMARY OF THE INVENTION**

It is an object of the present invention to provide a novel antibody or antigen-binding fragment thereof against ROR1 (receptor tyrosine kinase like orphan receptor 1).

It is another object of the present invention to provide a nucleic acid encoding the antibody or antigen-binding fragment thereof.

It is still another object of the present invention to provide a recombinant expression vector comprising the nucleic acid or a host cell transfected with the recombinant expression vector.

It is yet another object of the present invention to provide a method of producing an antibody or antigen-binding fragment thereof that specifically binds to ROR1.

It is still yet another object of the present invention to provide a bispecific/multispecific antibody or an immune cell-engaging bispecific/multispecific antibody comprising the antibody or antigen-binding fragment thereof.

It is even still another object of the present invention to provide an antibody-drug conjugate (ADC) in which the antibody or antigen-binding fragment thereof is conjugated to a drug.

It is even yet another object of the present invention to provide a chimeric antigen receptor (CAR) comprising an scFv of the antibody as an antigen-binding site of an extracellular domain, an immune cell into which the chimeric antigen receptor is introduced, and a composition for combination therapy comprising the immune cell.

It is a further object of the present invention to provide a composition for preventing or treating cancer or a method of preventing or treating cancer, comprising the antibody or antigen-binding fragment thereof, the bispecific or multispecific antibody, the antibody-drug conjugate, the chimeric antigen receptor, or the immune cell.

It is still a further object of the present invention to provide the use of the antibody or antigen-binding fragment thereof, the bispecific or multispecific antibody, the antibody-drug conjugate, the chimeric antigen receptor, or the immune cell for the prevention or treatment of cancer.

It is yet a further object of the present invention to provide the use of the antibody or antigen-binding fragment thereof, the bispecific or multispecific antibody, the antibody-drug conjugate, the chimeric antigen receptor, or the immune cell for the manufacture of a medicament for preventing or treating cancer.

In order to accomplish the above objects, the present invention provides an antibody or antigen-binding fragment thereof that specifically binds to ROR1 (receptor tyrosine kinase like orphan receptor 1), comprising:

a heavy chain CDR1 comprising at least one amino acid sequence selected from the group consisting of SEQ ID NO: 1 to SEQ ID NO: 16,
a heavy chain CDR2 comprising at least one amino acid sequence selected from the group consisting of SEQ ID NO: 17 to SEQ ID NO: 41, SEQ ID NO: 184, and SEQ ID NO: 185,
a heavy chain CDR3 comprising at least one amino acid sequence selected from the group consisting of SEQ ID NO: 42 to SEQ ID NO: 65,
a light chain CDR1 comprising at least one amino acid sequence selected from the group consisting of SEQ ID NO: 66 to SEQ ID NO: 86,
a light chain CDR2 comprising at least one amino acid sequence selected from the group consisting of SEQ ID NO: 87 to SEQ ID NO: 102, SEQ ID NO: 186, and SEQ ID NO: 187, and
a light chain CDR3 comprising at least one amino acid sequence selected from the group consisting of SEQ ID NO: 103 to SEQ ID NO: 121.

The present invention also provides a nucleic acid encoding the antibody or antigen-binding fragment thereof.

The present invention also provides a recombinant expression vector comprising the nucleic acid.

The present invention also provides a host cell transfected with the recombinant expression vector.

The present invention also provides a method of producing an antibody or antigen-binding fragment thereof that specifically binds to ROR1, comprising producing an antibody by culturing the host cell; and isolating and purifying the produced antibody.

The present invention also provides a bispecific/multispecific antibody or an immune cell-engaging bispecific/multispecific antibody comprising the antibody or antigen-binding fragment thereof.

The present invention also provides an antibody-drug conjugate (ADC) in which the antibody or antigen-binding fragment thereof is conjugated to a drug.

The present invention also provides a chimeric antigen receptor (CAR) comprising an extracellular domain comprising an antigen-binding site, a transmembrane domain, and an intracellular signaling domain, in which the antigen-binding site of the extracellular domain is an scFv of the antibody.

The present invention also provides an immune cell comprising the chimeric antigen receptor (CAR).

The present invention also provides a composition for preventing or treating cancer, comprising the antibody or antigen-binding fragment thereof, the bispecific or multispecific antibody, the antibody-drug conjugate, the chimeric antigen receptor, or the immune cell.

The present invention also provides the use of the antibody or antigen-binding fragment thereof, the bispecific or multispecific antibody, the antibody-drug conjugate, the chimeric antigen receptor, or the immune cell for the prevention or treatment of cancer.

The present invention also provides the use of the antibody or antigen-binding fragment thereof, the bispecific or multispecific antibody, the antibody-drug conjugate, the chimeric antigen receptor, or the immune cell for the manufacture of a medicament for preventing or treating cancer.

## BRIEF DESCRIPTION OF THE DRAWINGS

FIG. 1 shows results of analysis of ROR1 binding properties depending on clones through ELISA.
FIG. 2 shows results confirming that anti-ROR1 antibodies cross-link to human ROR1 and mouse ROR1.

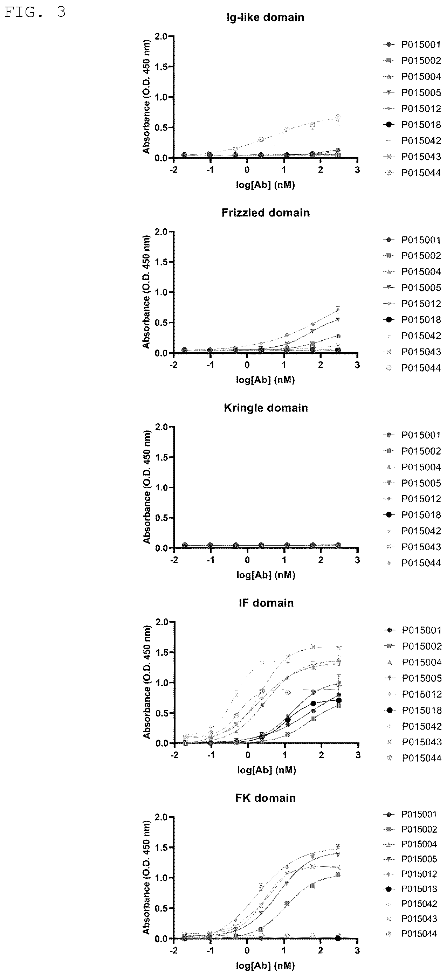

FIG. 3 shows results of analysis of anti-ROR1 antibody clones binding to individual domains through ELISA.
FIG. 4 shows results of measurement of ROR1 expression levels of cells used in Example of the present invention.
FIG. 5 shows results of analysis of the binding ability of antibodies used in Example of the present invention to an ROR1 antigen expressed in AMB-LC-0003T patient-derived lung cancer cells.
FIG. 6 shows results of analysis of the binding ability of the antibodies used in Example of the present invention to an ROR1 antigen expressed in a Jeko-1 cell line.
FIG. 7 shows results of cellular internalization analysis of the antibodies used in Example of the present invention.
FIG. 8 shows the structure of an anti-ROR1 antibody-drug conjugate.
FIG. 9 shows results of purity analysis of antibody-drug conjugates used in Example of the present invention.
FIG. 10 shows results of analysis of the average number of conjugated drug molecules per antibody molecule of the antibody-drug conjugate used in Example of the present invention.
FIG. 11 shows results of ELISA for ROR1 binding of the antibody-drug conjugates used in Example of the present invention.
FIG. 12 shows results of evaluation of *in vitro* toxicity of the antibody-drug conjugates used in Example of the present invention on patient-derived cells.
FIG. 13 shows results of evaluation of *in vitro* toxicity of the antibody-drug conjugates used in Example of the present invention on patient-derived cells, confirming greatly increased efficacy compared to a conventional antibody-drug conjugate.

## DETAILED DESCRIPTION OF THE INVENTION AND PREFERRED EMBODIMENTS OF THE INVENTION

Unless otherwise defined, all technical and scientific terms used herein have the same meanings as typically understood by those skilled in the art to which the present invention belongs. In general, the nomenclature used herein is well known in the art and is typical.

Anti-ROR1 antibody

An aspect of the present invention relates to an antibody or antigen-binding fragment thereof that specifically binds to ROR1 (receptor tyrosine kinase like orphan receptor 1), comprising a heavy chain CDR1 comprising at least one amino acid sequence selected from the group consisting of SEQ ID NO: 1 to SEQ ID NO: 16, a heavy chain CDR2 comprising at least one amino acid sequence selected from the group consisting of SEQ ID NO: 17 to SEQ ID NO: 41, SEQ ID NO: 184, and SEQ ID NO: 185, a heavy chain CDR3 comprising at least one amino acid sequence selected from the group consisting of SEQ ID NO: 42 to SEQ ID NO: 65, a light chain CDR1 comprising at least one amino acid sequence selected from the group consisting of SEQ ID NO: 66 to SEQ ID NO: 86, a light chain CDR2 comprising at least one amino acid sequence selected from the group consisting of SEQ ID NO: 87 to SEQ ID NO: 102, SEQ ID NO: 186, and SEQ ID NO: 187, and a light chain CDR3 comprising at least one amino acid sequence selected from the group consisting of SEQ ID NO: 103 to SEQ ID NO: 121.

As used herein, the term "antibody" refers to an anti-ROR1 antibody that specifically binds to ROR1. The scope of the present invention includes not only a complete antibody form that specifically binds to ROR1, but also antigen-binding fragments of the antibody molecule.

A complete antibody has a structure of two full-length light chains and two full-length heavy chains, with each light chain connected to the heavy chain by a disulfide bond.

As used herein, the term "heavy chain" is understood to comprise a full-length heavy chain and fragments thereof, comprising a variable region domain VH comprising an amino acid sequence having a variable region sequence sufficient to confer specificity to an antigen and three constant region domains CH1, CH2, and CH3. Also, the term "light chain" used herein is understood to comprise a full-length light chain and fragments thereof, comprising a variable region domain VL comprising an amino acid sequence having a variable region sequence sufficient to confer specificity to an antigen and a constant region domain CL.

The whole antibody comprises subtypes of IgA, IgD, IgE, IgM, and IgG, and, in particular, IgG includes IgG1, IgG2, IgG3, and IgG4. The heavy chain constant region has gamma ($\gamma$), mu ($\mu$), alpha ($\alpha$), delta ($\delta$), and epsilon ($\varepsilon$) types,

and gamma 1 ($\gamma$1), gamma 2 ($\gamma$2), gamma 3 ($\gamma$3), gamma 4 ($\gamma$4), alpha 1 ($\alpha$1), and alpha 2 ($\alpha$2) subclasses. The light chain constant region has kappa ($\kappa$) and lambda ($\lambda$) types.

The antigen-binding fragment of an antibody or the antibody fragment is a fragment having an antigen-binding function, and includes Fab, F(ab'), F(ab')$_2$, and Fv. Among antibody fragments, Fab has a structure having light and heavy chain variable regions, a light chain constant region, and a first heavy chain constant region (CH1), and has one antigen-binding site. Fab' differs from Fab in that Fab' has a hinge region comprising at least one cysteine residue at the C-terminus of the heavy chain CH1 domain. F(ab')$_2$ is produced by a disulfide bond between cysteine residues in the hinge region of Fab'.

Fv corresponds to a minimal antibody fragment having only a heavy chain variable region and a light chain variable region. A two-chain Fv is configured such that a heavy chain variable region and a light chain variable region are linked by a noncovalent bond, and a single-chain Fv (scFv) is configured such that a heavy chain variable region and a light chain variable region are generally linked by a covalent bond via a peptide linker therebetween, or are directly linked at the C-terminus, forming a dimeric structure, like the two-chain Fv. Such antibody fragments may be obtained using proteolytic enzymes (e.g., Fab may be obtained by restriction-cleaving a whole antibody with papain, and F(ab')$_2$ may be obtained by cleavage with pepsin), or may be constructed through genetic recombination technology.

An "Fv" fragment is an antibody fragment that contains a complete antibody recognition and binding site. This region is a dimer in which one heavy chain variable domain and one light chain variable domain are joined.

A "Fab" fragment includes the variable and constant domains of a light chain and the variable and first constant domains (CH1) of a heavy chain. F(ab')$_2$ antibody fragments generally include a pair of Fab' fragments covalently linked by cysteine in the hinge region present at the C-terminus of the Fab' fragment.

A "single-chain Fv (scFv)" antibody fragment is a construct composed of a single polypeptide chain including the VH and VL domains of an antibody. A polypeptide linker may be further included between the VH domain and the VL domain to enable scFv to form a desired structure for antigen binding.

In one embodiment, examples of the antibody of the present invention incldue, but are not limited to, monoclonal antibodies, multispecific antibodies, human antibodies, humanized antibodies, chimeric antibodies, scFvs, Fab fragments, F(ab')$_2$ fragments, disulfide-linked Fvs (sdFvs), anti-idiotype (anti-Id) antibodies, epitope-binding fragments of these antibodies, etc.

The heavy chain constant region may be any one selected from among isotypes such as gamma ($\gamma$), mu ($\mu$), alpha ($\alpha$), delta ($\delta$), and epsilon ($\varepsilon$). For example, the constant region is gamma 1 (IgG1), gamma 2 (IgG2), gamma 3 (IgG3), or gamma 4 (IgG4). The light chain constant region may be kappa or lambda type.

The monoclonal antibody is an antibody obtained from a population of substantially homogeneous antibodies, in which the individual antibodies that make up the population are identical, except for possible naturally-occurring mutations that may be present in minor amounts. A monoclonal antibody is highly specific and is directed against a single antigenic site. In contrast to typical (polyclonal) antibodies, which commonly include different antibodies directed against different determinants (epitopes), each monoclonal antibody is directed against a single determinant on the antigen.

The term "epitope" refers to a protein determinant to which an antibody is able to specifically bind. The epitope is usually composed of a group of chemically active surface molecules, for example amino acids or sugar side-chains, and generally has specific three-dimensional structural features and specific charge properties. Conformational and nonconformational epitopes are distinguished in that binding to the former, but not the latter, is lost in the presence of a denaturing solvent.

A non-human (e.g., mouse) antibody in "humanized" form is a chimeric antibody that contains a minimal sequence derived from a non-human immunoglobulin. In most cases, the humanized antibody is a human immunoglobulin (recipient antibody), in which a residue from the hypervariable region of a recipient is replaced with a residue from the hypervariable region of a non-human species (donor antibody) having the desired specificity, affinity, and capability, for example, mice, rats, rabbits, or non-human primates.

A "human antibody" is a molecule derived from human immunoglobulin, and means that all of the amino acid sequences constituting the antibody including a complementarity-determining region and a framework region are composed of human immunoglobulin.

A portion of the heavy chain and/or light chain is identical to or homologous with the corresponding sequence in an antibody derived from a particular species or belonging to a particular antibody class or subclass, whereas the remaining chain(s) includes a "chimeric" antibody (immunoglobulin) that is identical to or homologous with the corresponding sequence in an antibody derived from another species or belonging to another antibody class or subclass, as well as fragments of such antibodies that exhibit the desired biological activity.

The "variable region" of the antibody as used herein is a light chain or heavy chain portion of an antibody molecule comprising the amino acid sequence of a complementarity-determining region (CDR; i.e., CDR1, CDR2, and CDR3) and a framework region (FR). VH refers to the variable domain of a heavy chain, and VL refers to the variable domain of a light chain.

The "complementarity-determining region" (CDR) is an amino acid residue of the antibody variable domain that is necessary for antigen binding. Each variable domain typically has three CDRs, identified as CDR1, CDR2, and CDR3.

A heavy chain variable region comprising the heavy chain CDR1 of SEQ ID NO: 1, the heavy chain CDR2 of SEQ ID NO: 17, and the heavy chain CDR3 of SEQ ID NO: 42, and a light chain variable region comprising the light chain CDR1 of SEQ ID NO: 66, the light chain CDR2 of SEQ ID NO: 87, and the light chain CDR3 of SEQ ID NO: 103,

a heavy chain variable region comprising the heavy chain CDR1 of SEQ ID NO: 2, the heavy chain CDR2 of SEQ ID NO: 18, and the heavy chain CDR3 of SEQ ID NO: 43, and a light chain variable region comprising the light chain CDR1 of SEQ ID NO: 67, the light chain CDR2 of SEQ ID NO: 88, and the light chain CDR3 of SEQ ID NO: 104,

a heavy chain variable region comprising the heavy chain CDR1 of SEQ ID NO: 3, the heavy chain CDR2 of SEQ ID NO: 19, and the heavy chain CDR3 of SEQ ID NO: 44, and a light chain variable region comprising the light chain CDR1 of SEQ ID NO: 67, the light chain CDR2 of SEQ ID NO: 88, and the light chain CDR3 of SEQ ID NO: 104,

a heavy chain variable region comprising the heavy chain CDR1 of SEQ ID NO: 4, the heavy chain CDR2 of SEQ ID NO: 20, and the heavy chain CDR3 of SEQ ID NO: 45, and a light chain variable region comprising the light chain CDR1 of SEQ ID NO: 68, the light chain CDR2 of SEQ ID NO: 89, and the light chain CDR3 of SEQ ID NO: 105,

a heavy chain variable region comprising the heavy chain CDR1 of SEQ ID NO: 2, the heavy chain CDR2 of SEQ ID NO: 18, and the heavy chain CDR3 of SEQ ID NO: 43, and a light chain variable region comprising the light chain CDR1 of SEQ ID NO: 69, the light chain CDR2 of SEQ ID NO: 90, and the light chain CDR3 of SEQ ID NO: 106,

a heavy chain variable region comprising the heavy chain CDR1 of SEQ ID NO: 5, the heavy chain CDR2 of SEQ ID NO: 21, and the heavy chain CDR3 of SEQ ID NO: 46, and a light chain variable region comprising the light chain CDR1 of SEQ ID NO: 67, the light chain CDR2 of SEQ ID NO: 88, and the light chain CDR3 of SEQ ID NO: 104,

a heavy chain variable region comprising the heavy chain CDR1 of SEQ ID NO: 6, the heavy chain CDR2 of SEQ ID NO: 22, and the heavy chain CDR3 of SEQ ID NO: 47, and a light chain variable region comprising the light chain CDR1 of SEQ ID NO: 70, the light chain CDR2 of SEQ ID NO: 91, and the light chain CDR3 of SEQ ID NO: 107,

a heavy chain variable region comprising the heavy chain CDR1 of SEQ ID NO: 5, the heavy chain CDR2 of SEQ ID NO: 17, and the heavy chain CDR3 of SEQ ID NO: 48, and a light chain variable region comprising the light chain CDR1 of SEQ ID NO: 67, the light chain CDR2 of SEQ ID NO: 88, and the light chain CDR3 of SEQ ID NO: 108,

a heavy chain variable region comprising the heavy chain CDR1 of SEQ ID NO: 7, the heavy chain CDR2 of SEQ ID NO: 23, and the heavy chain CDR3 of SEQ ID NO: 49, and a light chain variable region comprising the light chain CDR1 of SEQ ID NO: 71, the light chain CDR2 of SEQ ID NO: 92, and the light chain CDR3 of SEQ ID NO: 109,

a heavy chain variable region comprising the heavy chain CDR1 of SEQ ID NO: 7, the heavy chain CDR2 of SEQ ID NO: 24, and the heavy chain CDR3 of SEQ ID NO: 50, and a light chain variable region comprising the light chain CDR1 of SEQ ID NO: 72, the light chain CDR2 of SEQ ID NO: 90, and the light chain CDR3 of SEQ ID NO: 109,

a heavy chain variable region comprising the heavy chain CDR1 of SEQ ID NO: 7, the heavy chain CDR2 of SEQ ID NO: 25, and the heavy chain CDR3 of SEQ ID NO: 47, and a light chain variable region comprising the light chain CDR1 of SEQ ID NO: 70, the light chain CDR2 of SEQ ID NO: 93, and the light chain CDR3 of SEQ ID NO: 108,

a heavy chain variable region comprising the heavy chain CDR1 of SEQ ID NO: 8, the heavy chain CDR2 of SEQ ID NO: 26, and the heavy chain CDR3 of SEQ ID NO: 51, and a light chain variable region comprising the light chain CDR1 of SEQ ID NO: 73, the light chain CDR2 of SEQ ID NO: 90, and the light chain CDR3 of SEQ ID NO: 110,

a heavy chain variable region comprising the heavy chain CDR1 of SEQ ID NO: 9, the heavy chain CDR2 of SEQ ID NO: 27, and the heavy chain CDR3 of SEQ ID NO: 52, and a light chain variable region comprising the light chain CDR1 of SEQ ID NO: 74, the light chain CDR2 of SEQ ID NO: 94, and the light chain CDR3 of SEQ ID NO: 111,

a heavy chain variable region comprising the heavy chain CDR1 of SEQ ID NO: 7, the heavy chain CDR2 of SEQ ID NO: 28, and the heavy chain CDR3 of SEQ ID NO: 50, and a light chain variable region comprising the light chain CDR1 of SEQ ID NO: 75, the light chain CDR2 of SEQ ID NO: 92, and the light chain CDR3 of SEQ ID NO: 112,

a heavy chain variable region comprising the heavy chain CDR1 of SEQ ID NO: 10, the heavy chain CDR2 of SEQ ID NO: 29, and the heavy chain CDR3 of SEQ ID NO: 53, and a light chain variable region comprising the light chain CDR1 of SEQ ID NO: 76, the light chain CDR2 of SEQ ID NO: 91, and the light chain CDR3 of SEQ ID NO: 113,

a heavy chain variable region comprising the heavy chain CDR1 of SEQ ID NO: 11, the heavy chain CDR2 of SEQ ID NO: 30, and the heavy chain CDR3 of SEQ ID NO: 54, and a light chain variable region comprising the light chain CDR1 of SEQ ID NO: 77, the light chain CDR2 of SEQ ID NO: 93, and the light chain CDR3 of SEQ ID NO: 114,

a heavy chain variable region comprising the heavy chain CDR1 of SEQ ID NO: 12, the heavy chain CDR2 of SEQ ID NO: 31, and the heavy chain CDR3 of SEQ ID NO: 55, and a light chain variable region comprising the light chain CDR1 of SEQ ID NO: 78, the light chain CDR2 of SEQ ID NO: 95, and the light chain CDR3 of SEQ ID NO: 115,

a heavy chain variable region comprising the heavy chain CDR1 of SEQ ID NO: 13, the heavy chain CDR2 of SEQ ID NO: 32, and the heavy chain CDR3 of SEQ ID NO: 56, and a light chain variable region comprising the light chain CDR1 of SEQ ID NO: 79, the light chain CDR2 of SEQ ID NO: 96, and the light chain CDR3 of SEQ ID NO: 116,

a heavy chain variable region comprising the heavy chain CDR1 of SEQ ID NO: 3, the heavy chain CDR2 of SEQ ID NO: 33, and the heavy chain CDR3 of SEQ ID NO: 57, and a light chain variable region comprising the light chain CDR1 of SEQ ID NO: 80, the light chain CDR2 of SEQ ID NO: 97, and the light chain CDR3 of SEQ ID NO: 114, a heavy chain variable region comprising the heavy chain CDR1 of SEQ ID NO: 1, the heavy chain CDR2 of SEQ ID NO: 34, and the heavy chain CDR3 of SEQ ID NO: 58, and a light chain variable region comprising the light chain CDR1 of SEQ ID NO: 81, the light chain CDR2 of SEQ ID NO: 98, and the light chain CDR3 of SEQ ID NO: 103, a heavy chain variable region comprising the heavy chain CDR1 of SEQ ID NO: 14, the heavy chain CDR2 of SEQ ID NO: 35, and the heavy chain CDR3 of SEQ ID NO: 59, and a light chain variable region comprising the light chain CDR1 of SEQ ID NO: 82, the light chain CDR2 of SEQ ID NO: 99, and the light chain CDR3 of SEQ ID NO: 106, a heavy chain variable region comprising the heavy chain CDR1 of SEQ ID NO: 15, the heavy chain CDR2 of SEQ ID NO: 36, and the heavy chain CDR3 of SEQ ID NO: 60, and a light chain variable region comprising the light chain CDR1 of SEQ ID NO: 76, the light chain CDR2 of SEQ ID NO: 91, and the light chain CDR3 of SEQ ID NO: 117, a heavy chain variable region comprising the heavy chain CDR1 of SEQ ID NO: 10, the heavy chain CDR2 of SEQ ID NO: 37, and the heavy chain CDR3 of SEQ ID NO: 61, and a light chain variable region comprising the light chain CDR1 of SEQ ID NO: 76, the light chain CDR2 of SEQ ID NO: 93, and the light chain CDR3 of SEQ ID NO: 118, a heavy chain variable region comprising the heavy chain CDR1 of SEQ ID NO: 7, the heavy chain CDR2 of SEQ ID NO: 23, and the heavy chain CDR3 of SEQ ID NO: 49, and a light chain variable region comprising the light chain CDR1 of SEQ ID NO: 71, the light chain CDR2 of SEQ ID NO: 92, and the light chain CDR3 of SEQ ID NO: 109, a heavy chain variable region comprising the heavy chain CDR1 of SEQ ID NO: 10, the heavy chain CDR2 of SEQ ID NO: 38, and the heavy chain CDR3 of SEQ ID NO: 62, and a light chain variable region comprising the light chain CDR1 of SEQ ID NO: 83, the light chain CDR2 of SEQ ID NO: 100, and the light chain CDR3 of SEQ ID NO: 109, a heavy chain variable region comprising the heavy chain CDR1 of SEQ ID NO: 7, the heavy chain CDR2 of SEQ ID NO: 23, and the heavy chain CDR3 of SEQ ID NO: 49, and a light chain variable region comprising the light chain CDR1 of SEQ ID NO: 71, the light chain CDR2 of SEQ ID NO: 92, and the light chain CDR3 of SEQ ID NO: 109, a heavy chain variable region comprising the heavy chain CDR1 of SEQ ID NO: 7, the heavy chain CDR2 of SEQ ID NO: 23, and the heavy chain CDR3 of SEQ ID NO: 49, and a light chain variable region comprising the light chain CDR1 of SEQ ID NO: 71, the light chain CDR2 of SEQ ID NO: 92, and the light chain CDR3 of SEQ ID NO: 119, a heavy chain variable region comprising the heavy chain CDR1 of SEQ ID NO: 10, the heavy chain CDR2 of SEQ ID NO: 39, and the heavy chain CDR3 of SEQ ID NO: 63, and a light chain variable region comprising the light chain CDR1 of SEQ ID NO: 84, the light chain CDR2 of SEQ ID NO: 101, and the light chain CDR3 of SEQ ID NO: 120, a heavy chain variable region comprising the heavy chain CDR1 of SEQ ID NO: 10, the heavy chain CDR2 of SEQ ID NO: 39, and the heavy chain CDR3 of SEQ ID NO: 63, and a light chain variable region comprising the light chain CDR1 of SEQ ID NO: 84, the light chain CDR2 of SEQ ID NO: 101, and the light chain CDR3 of SEQ ID NO: 109, a heavy chain variable region comprising the heavy chain CDR1 of SEQ ID NO: 1, the heavy chain CDR2 of SEQ ID NO: 40, and the heavy chain CDR3 of SEQ ID NO: 64, and a light chain variable region comprising the light chain CDR1 of SEQ ID NO: 85, the light chain CDR2 of SEQ ID NO: 97, and the light chain CDR3 of SEQ ID NO: 108, a heavy chain variable region comprising the heavy chain CDR1 of SEQ ID NO: 16, the heavy chain CDR2 of SEQ ID NO: 41, and the heavy chain CDR3 of SEQ ID NO: 65, and a light chain variable region comprising the light chain CDR1 of SEQ ID NO: 86, the light chain CDR2 of SEQ ID NO: 102, and the light chain CDR3 of SEQ ID NO: 121, a heavy chain variable region comprising the heavy chain CDR1 of SEQ ID NO: 10, the heavy chain CDR2 of SEQ ID NO: 184, and the heavy chain CDR3 of SEQ ID NO: 63, and a light chain variable region comprising the light chain CDR1 of SEQ ID NO: 84, the light chain CDR2 of SEQ ID NO: 101, and the light chain CDR3 of SEQ ID NO: 109, a heavy chain variable region comprising the heavy chain CDR1 of SEQ ID NO: 10, the heavy chain CDR2 of SEQ ID NO: 185, and the heavy chain CDR3 of SEQ ID NO: 63, and a light chain variable region comprising the light chain CDR1 of SEQ ID NO: 84, the light chain CDR2 of SEQ ID NO: 101, and the light chain CDR3 of SEQ ID NO: 109, a heavy chain variable region comprising the heavy chain CDR1 of SEQ ID NO: 10, the heavy chain CDR2 of SEQ ID NO: 39, and the heavy chain CDR3 of SEQ ID NO: 63, and a light chain variable region comprising the light chain CDR1 of SEQ ID NO: 84, the light chain CDR2 of SEQ ID NO: 186, and the light chain CDR3 of SEQ ID NO: 109, a heavy chain variable region comprising the heavy chain CDR1 of SEQ ID NO: 10, the heavy chain CDR2 of SEQ ID NO: 39, and the heavy chain CDR3 of SEQ ID NO: 63, and a light chain variable region comprising the light chain CDR1 of SEQ ID NO: 84, the light chain CDR2 of SEQ ID NO: 187, and the light chain CDR3 of SEQ ID NO: 109, a heavy chain variable region comprising the heavy chain CDR1 of SEQ ID NO: 10, the heavy chain CDR2 of SEQ ID NO: 39, and the heavy chain CDR3 of SEQ ID NO: 63, and a light chain variable region comprising the light chain CDR1 of SEQ ID NO: 84, the light chain CDR2 of SEQ ID NO: 101, and the light chain CDR3 of SEQ ID NO: 110, or a heavy chain variable region comprising the heavy chain CDR1 of SEQ ID NO: 10, the heavy chain CDR2 of SEQ ID NO: 185, and the heavy chain CDR3 of SEQ ID NO: 63, and a light chain variable region comprising the light chain CDR1 of SEQ ID NO: 84, the light chain CDR2 of SEQ ID NO: 187, and the light chain CDR3 of SEQ ID NO: 110.

The "framework region" (FR) is a variable domain residue other than the CDR residue. Each variable domain typically has four FRs, identified as FR1, FR2, FR3, and FR4.

The binding affinity of the anti-ROR1 antibody to ROR1 falls in the range of $10^{-5}$ M to $10^{-12}$ M. For example, the binding affinity of the anti-ROR1 antibody to ROR1 is $10^{-6}$ M to $10^{-12}$ M, $10^{-7}$ M to $10^{-12}$ M, $10^{-8}$ M to $10^{-12}$ M, $10^{-9}$ M to $10^{-12}$ M, $10^{-5}$ M to $10^{-11}$ M, $10^{-6}$ M to $10^{-11}$ M, $10^{-7}$ M to $10^{-11}$ M, $10^{-8}$ M to $10^{-11}$ M, $10^{-9}$ M to $10^{-11}$ M, $10^{-10}$ M to $10^{-11}$ M, $10^{-5}$ M to $10^{-10}$ M, $10^{-6}$ M to $10^{-10}$ M, $10^{-7}$ M to $10^{-10}$ M, $10^{-8}$ M to $10^{-10}$ M, $10^{-9}$ M to $10^{-10}$ M, $10^{-5}$ M to $10^{-9}$ M, $10^{-6}$ M to $10^{-9}$ M, $10^{-7}$ M to $10^{-9}$ M, $10^{-8}$ M to $10^{-9}$ M, $10^{-5}$ M to $10^{-8}$ M, $10^{-6}$ M to $10^{-8}$ M, $10^{-7}$ M to $10^{-8}$ M, $10^{-5}$ M to $10^{-7}$ M, $10^{-6}$ M to $10^{-7}$ M, or $10^{-5}$ M to $10^{-6}$ M.

The antibody or antigen-binding fragment thereof that binds to ROR1 may comprise a heavy chain variable region comprising at least one amino acid sequence selected from the group consisting of SEQ ID NO: 122 to SEQ ID NO: 152 and SEQ ID NO: 188 to SEQ ID NO: 193. Additionally, the antibody or antigen-binding fragment thereof that binds to ROR1 may comprise a light chain variable region comprising at least one amino acid sequence selected from the group consisting of SEQ ID NO: 153 to SEQ ID NO: 183 and SEQ ID NO: 194 to SEQ ID NO: 199.

In a specific embodiment according to the present invention, the antibody or antigen-binding fragment thereof may comprise:

the heavy chain variable region of SEQ ID NO: 122 and the light chain variable region of SEQ ID NO: 153;
the heavy chain variable region of SEQ ID NO: 123 and the light chain variable region of SEQ ID NO: 154;
the heavy chain variable region of SEQ ID NO: 124 and the light chain variable region of SEQ ID NO: 155;
the heavy chain variable region of SEQ ID NO: 125 and the light chain variable region of SEQ ID NO: 156;
the heavy chain variable region of SEQ ID NO: 126 and the light chain variable region of SEQ ID NO: 157;
the heavy chain variable region of SEQ ID NO: 127 and the light chain variable region of SEQ ID NO: 158;
the heavy chain variable region of SEQ ID NO: 128 and the light chain variable region of SEQ ID NO: 159;
the heavy chain variable region of SEQ ID NO: 129 and the light chain variable region of SEQ ID NO: 160;
the heavy chain variable region of SEQ ID NO: 130 and the light chain variable region of SEQ ID NO: 161;
the heavy chain variable region of SEQ ID NO: 131 and the light chain variable region of SEQ ID NO: 162;
the heavy chain variable region of SEQ ID NO: 132 and the light chain variable region of SEQ ID NO: 163;
the heavy chain variable region of SEQ ID NO: 133 and the light chain variable region of SEQ ID NO: 164;
the heavy chain variable region of SEQ ID NO: 134 and the light chain variable region of SEQ ID NO: 165;
the heavy chain variable region of SEQ ID NO: 135 and the light chain variable region of SEQ ID NO: 166;
the heavy chain variable region of SEQ ID NO: 136 and the light chain variable region of SEQ ID NO: 167;
the heavy chain variable region of SEQ ID NO: 137 and the light chain variable region of SEQ ID NO: 168;
the heavy chain variable region of SEQ ID NO: 138 and the light chain variable region of SEQ ID NO: 169;
the heavy chain variable region of SEQ ID NO: 139 and the light chain variable region of SEQ ID NO: 170;
the heavy chain variable region of SEQ ID NO: 140 and the light chain variable region of SEQ ID NO: 171;
the heavy chain variable region of SEQ ID NO: 141 and the light chain variable region of SEQ ID NO: 172;
the heavy chain variable region of SEQ ID NO: 142 and the light chain variable region of SEQ ID NO: 173;
the heavy chain variable region of SEQ ID NO: 143 and the light chain variable region of SEQ ID NO: 174;
the heavy chain variable region of SEQ ID NO: 144 and the light chain variable region of SEQ ID NO: 175;
the heavy chain variable region of SEQ ID NO: 145 and the light chain variable region of SEQ ID NO: 176;
the heavy chain variable region of SEQ ID NO: 146 and the light chain variable region of SEQ ID NO: 177;
the heavy chain variable region of SEQ ID NO: 147 and the light chain variable region of SEQ ID NO: 178;
the heavy chain variable region of SEQ ID NO: 148 and the light chain variable region of SEQ ID NO: 179;
the heavy chain variable region of SEQ ID NO: 149 and the light chain variable region of SEQ ID NO: 180;
the heavy chain variable region of SEQ ID NO: 150 and the light chain variable region of SEQ ID NO: 181;
the heavy chain variable region of SEQ ID NO: 151 and the light chain variable region of SEQ ID NO: 182;
the heavy chain variable region of SEQ ID NO: 152 and the light chain variable region of SEQ ID NO: 183;
the heavy chain variable region of SEQ ID NO: 188 and the light chain variable region of SEQ ID NO: 194;
the heavy chain variable region of SEQ ID NO: 189 and the light chain variable region of SEQ ID NO: 195;
the heavy chain variable region of SEQ ID NO: 190 and the light chain variable region of SEQ ID NO: 196;
the heavy chain variable region of SEQ ID NO: 191 and the light chain variable region of SEQ ID NO: 197;
the heavy chain variable region of SEQ ID NO: 192 and the light chain variable region of SEQ ID NO: 198; or
the heavy chain variable region of SEQ ID NO: 193 and the light chain variable region of SEQ ID NO: 199.

<u>scFv</u>

An scFv is an antibody fragment, which is composed of a single polypeptide chain comprising the VH and VL domains of an antibody. A polypeptide linker may be additionally included between the VH domain and the VL domain to enable scFv to form a desired structure for antigen binding.

In one embodiment, in a single-chain Fv (scFv) comprising the VH and VL domains of an antibody, the VH and VL domains may be connected via a linker. A heavy chain variable region comprising at least one amino acid sequence selected from the group consisting of SEQ ID NO: 122 to SEQ ID NO: 152 and SEQ ID NO: 188 to SEQ ID NO: 193 may be connected to a light chain variable region comprising at least one amino acid sequence selected from the group consisting of SEQ ID NO: 153 to SEQ ID NO: 183 and SEQ ID NO: 194 to SEQ ID NO: 199 via a linker.

In a specific embodiment according to the present invention, the scFv may comprise:

the heavy chain variable region of SEQ ID NO: 122 and the light chain variable region of SEQ ID NO: 153;
the heavy chain variable region of SEQ ID NO: 123 and the light chain variable region of SEQ ID NO: 154;
the heavy chain variable region of SEQ ID NO: 124 and the light chain variable region of SEQ ID NO: 155;
the heavy chain variable region of SEQ ID NO: 125 and the light chain variable region of SEQ ID NO: 156;
the heavy chain variable region of SEQ ID NO: 126 and the light chain variable region of SEQ ID NO: 157;
the heavy chain variable region of SEQ ID NO: 127 and the light chain variable region of SEQ ID NO: 158;
the heavy chain variable region of SEQ ID NO: 128 and the light chain variable region of SEQ ID NO: 159;
the heavy chain variable region of SEQ ID NO: 129 and the light chain variable region of SEQ ID NO: 160;
the heavy chain variable region of SEQ ID NO: 130 and the light chain variable region of SEQ ID NO: 161;
the heavy chain variable region of SEQ ID NO: 131 and the light chain variable region of SEQ ID NO: 162;
the heavy chain variable region of SEQ ID NO: 132 and the light chain variable region of SEQ ID NO: 163;
the heavy chain variable region of SEQ ID NO: 133 and the light chain variable region of SEQ ID NO: 164;
the heavy chain variable region of SEQ ID NO: 134 and the light chain variable region of SEQ ID NO: 165;
the heavy chain variable region of SEQ ID NO: 135 and the light chain variable region of SEQ ID NO: 166;
the heavy chain variable region of SEQ ID NO: 136 and the light chain variable region of SEQ ID NO: 167;
the heavy chain variable region of SEQ ID NO: 137 and the light chain variable region of SEQ ID NO: 168;
the heavy chain variable region of SEQ ID NO: 138 and the light chain variable region of SEQ ID NO: 169;
the heavy chain variable region of SEQ ID NO: 139 and the light chain variable region of SEQ ID NO: 170;
the heavy chain variable region of SEQ ID NO: 140 and the light chain variable region of SEQ ID NO: 171;
the heavy chain variable region of SEQ ID NO: 141 and the light chain variable region of SEQ ID NO: 172;
the heavy chain variable region of SEQ ID NO: 142 and the light chain variable region of SEQ ID NO: 173;
the heavy chain variable region of SEQ ID NO: 143 and the light chain variable region of SEQ ID NO: 174;
the heavy chain variable region of SEQ ID NO: 144 and the light chain variable region of SEQ ID NO: 175;
the heavy chain variable region of SEQ ID NO: 145 and the light chain variable region of SEQ ID NO: 176;
the heavy chain variable region of SEQ ID NO: 146 and the light chain variable region of SEQ ID NO: 177;
the heavy chain variable region of SEQ ID NO: 147 and the light chain variable region of SEQ ID NO: 178;
the heavy chain variable region of SEQ ID NO: 148 and the light chain variable region of SEQ ID NO: 179;
the heavy chain variable region of SEQ ID NO: 149 and the light chain variable region of SEQ ID NO: 180;
the heavy chain variable region of SEQ ID NO: 150 and the light chain variable region of SEQ ID NO: 181;
the heavy chain variable region of SEQ ID NO: 151 and the light chain variable region of SEQ ID NO: 182;
the heavy chain variable region of SEQ ID NO: 152 and the light chain variable region of SEQ ID NO: 183;
the heavy chain variable region of SEQ ID NO: 188 and the light chain variable region of SEQ ID NO: 194;
the heavy chain variable region of SEQ ID NO: 189 and the light chain variable region of SEQ ID NO: 195;
the heavy chain variable region of SEQ ID NO: 190 and the light chain variable region of SEQ ID NO: 196;
the heavy chain variable region of SEQ ID NO: 191 and the light chain variable region of SEQ ID NO: 197;
the heavy chain variable region of SEQ ID NO: 192 and the light chain variable region of SEQ ID NO: 198; or
the heavy chain variable region of SEQ ID NO: 193 and the light chain variable region of SEQ ID NO: 199.

The linker may be a peptide linker and may be about 10-25 aa long. Examples of the linker may comprise, but are not limited to, hydrophilic amino acids such as glycine and/or serine.

Specifically, the linker may comprise, for example, $(GS)_n$, $(GGS)_n$, $(GSGGS)_n$, or $(G_nS)_m$ (in which each of n and m is 1 to 10), but the linker may be, for example, $(G_nS)_m$ (in which each of n and m is 1 to 10). Specifically, the linker may comprise GGGGS, for example, GGGGSGGGGSGGGGS of SEQ ID NO: 200 repeated three times.

"Phage display" is a technique for displaying a variant polypeptide as a fusion protein with at least a portion of an envelope protein on the surface of a phage, for example, a filamentous phage particle. The usefulness of phage display lies in the fact that it may quickly and efficiently classify sequences that bind to target antigens with high affinity in large libraries of randomized protein variants. Displaying peptide and protein libraries on phages has been used to screen millions of polypeptides to identify polypeptides with specific binding properties.

Phage display technology provides a powerful tool for producing and screening novel proteins that bind to specific ligands (e.g., antigens). Using phage display technology, large libraries of protein variants may be constructed and sequences that bind with high affinity to target antigens may be rapidly sorted. A nucleic acid encoding a variant polypeptide is fused with a nucleic acid sequence encoding a viral envelope protein, such as a gene III protein or a gene VIII protein. A monovalent phage display system has been developed in which a nucleic acid sequence encoding a protein or polypeptide is fused with a nucleic acid sequence encoding a portion of a gene III protein. In the monovalent phage display system, the gene fusion is expressed at a low level and the wild-type gene III protein is also expressed, maintaining particle infectivity.

Demonstrating the expression of peptides on the filamentous phage surface and the expression of functional antibody fragments in the periplasm of *E. coli* is important in developing antibody phage display libraries. Libraries of antibodies or antigen-binding polypeptides have been constructed in a number of ways, for example, by altering a single gene by insertion of random DNA sequences or by cloning a family of relevant genes. The libraries may be screened for expression of antibodies or antigen-binding proteins with desired characteristics.

Phage display technology has several advantages over typical hybridoma and recombinant methods for producing antibodies with desired characteristics. This technology allows the construction of large antibody libraries with diverse sequences in a short period of time without using animals. The production of hybridomas or humanized antibodies may require several months of production time. Furthermore, since no immunity is required, phage antibody libraries may be used to produce antibodies even against toxic or low immunogenicity antigens. Phage antibody libraries may also be used to produce and identify new therapeutic antibodies.

Techniques for producing human antibodies from immunized or non-immunized human, germline sequences, or naive B cell Ig repertoires using phage display libraries may be used. Naive or non-immune antigen-binding libraries may be constructed using various lymphatic tissues.

Technology capable of identifying and isolating high-affinity antibodies from phage display libraries is important for isolating new therapeutic antibodies. Isolation of high-affinity antibodies from the libraries may depend on the size of the libraries, the efficiency of production in bacterial cells, and the diversity of the libraries. The size of the libraries is reduced by inefficient production due to the presence of stop codons and improper folding of the antibody or antigen-binding protein. Expression in bacterial cells may be inhibited if the antibody or antigen-binding domain is not properly folded. Expression may be improved by alternately mutating residues on the surface of the variable/constant interface or in selected CDR residues. The sequence of the framework region is an element for providing proper folding when constructing antibody phage libraries in bacterial cells.

In high-affinity antibody isolation, it is important to construct diverse libraries of antibodies or antigen-binding proteins. CDR3 regions have been found to often participate in antigen binding. Since the CDR3 region on the heavy chain varies considerably in size, sequence, and structural conformation, various libraries may be constructed using the same.

Also, diversity may be generated by randomizing the CDR regions of the variable heavy and light chains using all 20 amino acids at each position. The use of all 20 amino acids may result in production of variant antibody sequences having high diversity and an increase in the opportunity to identify new antibodies.

The antibody or antibody fragment according to the present invention may comprise not only the sequence of the anti-ROR1 antibody of the present invention described herein, but also biological equivalents thereto, to the extent that it may specifically recognize ROR1. For example, additional modifications may be made to the amino acid sequence of an antibody in order to further improve the binding affinity and/or other biological properties of the antibody. Such modifications include, for example, deletion, insertion, and/or substitution of the amino acid sequence residues of the antibody. The amino acid variations are based on the relative similarity of amino acid side-chain substituents with regard to, for example, hydrophobicity, hydrophilicity, charge, size, and the like. Based on analysis of the size, shape, and type of amino acid side-chain substituents, all of arginine, lysine, and histidine are positively charged residues, alanine, glycine, and serine have similar sizes, and phenylalanine, tryptophan, and tyrosine have similar shapes. Therefore, based on these considerations, arginine, lysine, and histidine may be regarded as biologically functional equivalents, alanine, glycine, and serine may be regarded as biologically functional equivalents, and phenylalanine, tryptophan, and tyrosine may be regarded as biologically functional equivalents.

Taking into consideration the above-described variations having equivalent biological activity, the antibody of the present invention or a nucleic acid molecule encoding the same is to be understood as comprising a sequence showing substantial identity to the sequence set forth in the sequence number. When the sequence of the present invention and any other sequences are aligned so as to correspond to each other as closely as possible and the aligned sequence is analyzed using an algorithm commonly used in the art, "substantial identity" refers to sequences exhibiting at least 90% homology, most preferably at least 95% homology, at least 96% homology, at least 97% homology, at least 98% homology, or at least 99% homology. Alignment methods for sequence comparison are known in the art. The NCBI Basic Local Alignment Search Tool (BLAST) is accessible through NCBI and elsewhere, and may be used in conjunction with sequencing programs such as blastp, blasm, blastx, tblastn, and tblastx on the Internet. BLAST is available at www.ncbi.nlm.nih.gov/BLAST/. A method for comparing sequence homology using this program may be found at www.ncbi.nlm.nih.gov/BLAST/blast_help.html.

Based thereon, the antibody or antigen-binding fragment thereof according to the present invention may have 90%, 91%, 92%, 93%, 94%, 95%, 96%, 97%, 98%, 99%, or higher homology with a specified sequence or all of the sequences set forth herein. Such homology may be determined through sequence comparison and/or alignment using methods known in the art. For example, the percentage sequence homology of a nucleic acid or protein according to the present invention may be determined using a sequence comparison algorithm (i.e., BLAST or BLAST 2.0), manual alignment, or visual inspection.

In another aspect, the present invention relates to a nucleic acid encoding the antibody or antigen-binding fragment thereof. An antibody or antigen-binding fragment thereof may be produced recombinantly by isolating the nucleic acid encoding the antibody or antigen-binding fragment thereof according to the present invention.

"Nucleic acid" has a meaning comprehensively encompassing DNA (gDNA and cDNA) and RNA molecules, and nucleotides, which are the basic building blocks of nucleic acids, include not only natural nucleotides but also analogues in which sugar or base regions are modified. The sequence of the nucleic acid encoding the heavy and light chain variable regions of the present invention may be modified. Such modification includes addition, deletion, or non-conservative or conservative substitution of nucleotides.

DNA encoding the antibody may be easily isolated or synthesized using typical molecular biological techniques (e.g., using an oligonucleotide probe capable of specifically binding to DNA encoding the heavy and light chains of the antibody). The nucleic acid is isolated and inserted into a replicable vector for further cloning (DNA amplification) or further expression. Based thereon, in still another aspect, the present invention relates to a recombinant expression vector comprising the nucleic acid.

As used herein, the term "vector" refers to a means for expressing a target gene in a host cell, and comprises a plasmid vector, a cosmid vector, a virus vector, such as a bacteriophage vector, an adenovirus vector, a retrovirus vector, and an adeno-associated virus vector, etc. Components of the vector generally include, but are not limited to, one or more selected from among the following: a signal sequence, an origin of replication, at least one antibiotic resistance marker gene, an enhancer element, a promoter, and a transcription termination sequence. The nucleic acid encoding the antibody is operably linked with a promoter and a transcription termination sequence.

"Operably linked" means a functional linkage between a nucleic acid expression control sequence (e.g., a promoter, a signal sequence, or an array of transcription regulator binding sites) and a different nucleic acid sequence, whereby the control sequence serves to control the transcription and/or translation of the different nucleic acid sequence.

When a prokaryotic cell is used as a host, a strong promoter capable of promoting transcription (e.g., a tac promoter, lac promoter, lacUV5 promoter, lpp promoter, pL$\lambda$ promoter, pR$\lambda$ promoter, rac5 promoter, amp promoter, recA promoter, SP6 promoter, trp promoter, or T7 promoter), a ribosome-binding site for initiation of translation, and a transcription/translation termination sequence are generally included. In addition, for example, when a eukaryotic cell is used as a host, a promoter derived from the genome of a mammalian cell (e.g., a metallothionine promoter, $\beta$-actin promoter, human hemoglobin promoter, or human muscle creatine promoter) or a promoter derived from a mammalian virus (e.g., an adenovirus late promoter, vaccinia virus 7.5k promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), or promoter of Rous sarcoma virus (RSV)) may be used, and a polyadenylation sequence is generally used as a transcription termination sequence.

In some cases, the vector may be fused with another sequence in order to facilitate purification of the antibody expressed therefrom. Examples of the sequence that is fused therewith include glutathione S-transferase (Pharmacia, USA), maltose-binding protein (NEB, USA), FLAG (IBI, USA), and 6x His (hexa-histidine; Qiagen, USA).

The vector comprises, as a selective marker, an antibiotic resistance gene that is commonly used in the art, for example, a gene conferring resistance to ampicillin, gentamicin, carbenicillin, chloramphenicol, streptomycin, kanamycin, geneticin, neomycin, or tetracycline.

In yet another aspect, the present invention relates to a host cell transfected with the recombinant expression vector. The host cell used to produce the antibody of the present invention may be, but are not limited to, prokaryotic cells, yeast cells, and higher eukaryotic cells.

Prokaryotic host cells, such as *Escherichia coli, Bacillus subtilis* and *Bacillus thuringiensis* as strains belonging to the genus *Bacillus, Streptomyces, Pseudomonas* (e.g., *Pseudomonas putida), Proteus mirabilis,* and *Staphylococcus* (e.g., *Staphylococcus carnosus),* may be used.

Here, animal cells are of greatest interest, and examples of useful host cell lines may include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In still yet another aspect, the present invention relates to a method of producing an antibody or antigen-binding fragment thereof that specifically binds to ROR1, comprising producing an antibody by culturing the host cell; and isolating and purifying the produced antibody.

The host cells may be cultured in various media. Any commercially available medium may be used as a culture medium without limitation. All other essential supplements known to those skilled in the art may be contained in appropriate concentrations. Culture conditions, such as temperature, pH, etc., are already in use with the host cells selected for expression, as will be apparent to those skilled in the art.

For the recovery of the antibody or antigen-binding fragment thereof, impurities may be removed by, for example, centrifugation or ultrafiltration, and the resultant product may be purified using, for example, affinity chromatography, etc. Other additional purification techniques, such as anion or cation exchange chromatography, hydrophobic interaction chromatography, hydroxyapatite chromatography, and the like, may be used.

Bispecific or multispecific antibody

In even still another aspect, the present invention relates to a bispecific or multispecific antibody comprising the antibody or antigen-binding fragment thereof.

A bispecific antibody is an antibody having the ability to bind to or antagonize one or more targets, and is configured such that an antibody having the ability to bind to or antagonize two different targets is bound or is an antibody in which an antibody having the ability to bind to one target is bound to a material having the ability to antagonize another target.

A multispecific antibody is an antibody having binding specificity to at least three different antigens. Multispecific antibodies may include tri- or higher specific antibodies, such as tri-specific antibodies, tetra-specific antibodies, or antibodies targeting more targets.

Antibodies belonging to bispecific or multispecific antibodies may be classified into scFv-based antibodies, Fab-based antibodies, and IgG-based antibodies. Bispecific or multispecific antibodies may inhibit or amplify two or more signals at the same time and thus may be more effective than when inhibiting/amplifying a single signal. Compared to cases in which signals are treated with respective signal inhibitors, low-dose administration is possible and two or more signals in the same time and space may be inhibited/amplified.

Methods of producing bispecific or multispecific antibodies are well known. Traditionally, recombinant production of bispecific antibodies is based on the co-expression of two or more immunoglobulin heavy/light chain pairs under conditions where two or more heavy chains have different specificities.

For scFv-based bispecific or multispecific antibodies, a diabody may be created by combining VL and VH of different scFvs to produce a hybrid scFv in heterodimeric form, a tandem scFv may be produced by connecting different scFvs to each other, a heterodimeric miniantibody may be produced by expressing CH1 and CL of Fab in the ends of each scFv, and a minibody in the form of a heterodimeric scFv may be produced by substituting some amino acids in the CH3 domain, which is a homodimeric domain of Fc, to change the same to a heterodimeric structure in the "knob-into-hole" form and expressing the changed CH3 domain in different ends of each scFv.

Fab-based bispecific or multispecific antibodies may be produced in the form of a heterodimeric Fab by combining Fab' fragments for a specific antigen with each other using a disulfide bond or a mediator, and may be produced to have two antigen valencies by expressing scFvs for different antigens in the end of the heavy or light chain of a specific Fab or to have four antigen valencies in homodimeric form by providing a hinge region between Fab and scFv. Also, there may be provided a double-targeting bibody having three antigen valencies by fusing scFvs for different antigens to the light and heavy chain ends of Fab, and a triple-targeting bibody having three antigen valencies by fusing different scFvs to the light and heavy chain ends of Fab, which may be obtained by chemically conjugating three different Fabs.

For IgG-based bispecific or multispecific antibodies, a method of producing bispecific antibodies by crossing mouse and rat hybridomas again to form hybrid hybridomas, so-called quadromas, has been disclosed by Trion Pharma. In addition, bispecific antibodies may be produced in the heterodimeric form, so-called "holes and knob" form, by modifying some amino acids of the CH3 homodimeric domain of Fc for different heavy chains while sharing the light chain portion. In addition to the heterodimeric bispecific antibodies, homodimeric (scFv)4-IgG may be produced by fusing and expressing two different scFvs to and in the constant domains instead of the variable domains of the light and heavy chains of IgG. Also, ImClone reported the production of a bispecific antibody based on IMC-1C11, a chimeric monoclonal antibody against human VEGFR-2, by fusing only the single variable domain for mouse platelet-derived growth factor receptor-$\alpha$ to the light chain amino terminus of this antibody. Also, the so-called "dock and lock (DNL)" method using the dimerization and docking domain (DDD) of the protein kinase A (PKA) R subunit and the anchoring domain of PKA enables the production of antibodies having multiple antigen valencies to CD20.

A wide variety of recombinant antibody formats have been developed, including at least divalent, trivalent, or tetravalent bispecific or multispecific antibodies. For example, International Patent Application Publication Nos. WO2001/077342, WO2009/080251, WO2009/080252, WO2009/080253, WO2009/080254, WO2010/112193, WO2010/115589, WO2010/136172, WO2010/145792, WO2010/145793, and WO2011/117330 disclose at least divalent, trivalent, or tetravalent antibodies. At least divalent, trivalent, or tetravalent antibody indicates that two or more binding domains, three or more binding domains, or four or more binding domains are present in the antibody molecule, respectively.

In a specific embodiment, the bispecific or multispecific antibody according to the present invention may comprise the anti-ROR1 antibody or antigen-binding fragment in the form of an IgG complete antibody or fragment thereof, for example, single-chain Fv, $V_H$ domain and/or $V_L$ domain, Fab, or (Fab)$_2$.

Also, an antibody binding to a different target from the antibody targeting ROR1, for example an antibody targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO, may be included in the form of an IgG complete antibody or fragment thereof, for example single-chain Fv, $V_H$ domain and/or $V_L$ domain, Fab, or (Fab)$_2$.

Through the bispecific or multispecific antibody according to the present invention, additional binding specificity induced or mediated by other targets in addition to ROR1 may be obtained.

For example, the bispecific antibody according to the present invention is capable of simultaneously targeting ROR1 and at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO.

For example, the multispecific antibody according to the present invention is capable of simultaneously targeting ROR1 and two or more selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO.

Immune cell-engaging bispecific or multispecific antibody

In even yet another aspect, the present invention relates to an immune cell-engaging bispecific or multispecific antibody comprising an scFv of the antibody and scFv comprised of a second binding domain comprising at least one scFv of an antibody binding to an immune cell-activating antigen.

The immune cell-engaging bispecific or multispecific antibody temporarily induces cytolytic synapses between cytotoxic T cells and cancer target cells to release toxins.

In one embodiment, the immune cell may be at least one selected from the group consisting of T cells, NK cells, cytokine induced killer cells (CIKs), activated cytotoxic T lymphocytes (CTLs), macrophages, tumor-infiltrating lymphocytes (TILs), and dendritic cells.

In one embodiment, the immune cell-activating antigen may be selected from among the following, and the antibody that binds thereto may serve as an immune cell engager:

a T cell-activating antigen such as CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
an NK cell-activating antigen such as NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160;
a B cell-activating antigen such as OX40, CD40, or CD70;
a macrophage-activating antigen such as CD2 agonist, CD40, CD70, TCR (toll-like receptor) agonist, CD47, STING, or OX40L; and
a dendritic cell-activating antigen such as a CD2 agonist, OX40, OX40L, 41BB agonist, TCR agonist, CD47 agonist, or STING agonist.

The immune cell engager is specified in U.S. Patent Application Publication No. 2017/0368169, which may be incorporated herein by reference.

Specifically, the immune cell-engaging bispecific or multispecific antibody may comprise a tandem scFv and may bind to the following antigen and surface antigen on cancer cells. Wherein, the surface antigen on the cancer cells is ROR1, targeted by the antibody according to the invention:

CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160;
OX40, CD40, or CD70;
CD2 agonist, CD40, CD70, TCR (toll-like receptor) agonist, CD47, STING, or OX40L; or
CD2 agonist, OX40, OX40L, 41BB agonist, TCR agonist, CD47 agonist, or STING agonist.

The immune cell-engaging bispecific or multispecific antibody may comprise, for example, a structure in the form of VL(ROR1)-VH(ROR1)-VH(CD3 or CD16A)-VL(CD3 or CD16A), VH(ROR1)-VL(ROR1)-VH(CD3 or CD16A)-VL(CD3 or CD16A), VH(CD3 or CD16A)-VL(CD3 or CD16A)-VH(ROR1)-VL(ROR1), or VH(CD3 or CD16A)-VL(CD3 or CD16A)-VL(ROR1)-VH(ROR1).

For example, the scFv may comprise a heavy chain variable region comprising at least one amino acid sequence selected from the group consisting of SEQ ID NO: 122 to SEQ ID NO: 152 and SEQ ID NO: 188 to SEQ ID NO: 193 and a light chain variable region comprising at least one amino acid sequence selected from the group consisting of SEQ ID NO: 153 to SEQ ID NO: 183 and SEQ ID NO: 194 to SEQ ID NO: 199, and the heavy chain variable region and the light chain variable region may be connected via a linker.

The linker may be a peptide linker and may have a length of about 10-25 aa. Examples of the linker may comprise hydrophilic amino acids such as glycine and/or serine.

The linker may include, for example, (GS)n, (GGS)n, (GSGGS)n, or (GnS)m (in which each of n and m is 1 to 10), but the linker may be, for example, $(G_nS)_m$ (in which each of n and m is 1 to 10). Specifically, the linker may include GGGGS, for example, GGGGSGGGGSGGGGS of SEQ ID NO: 200 repeated three times.

Examples of the immune cell-engaging bispecific or multispecific antibody may include blinatumomab (Amgen) binding to CD3 and CD19, solitomab (Amgen) binding to CD3 and EpCAM, MEDI 565 (MedImmune, Amgen) binding to CD3 and CEA, and BAY2010112 (Bayer, Amgen) binding to CD3 and PSMA. Examples of DART include MGD006 (Macrogenics) binding CD3 and CD123 and MGD007 (Macrogenics) binding to CD3 and gpA33. Examples of TandAbs may include AFM11 (Affimed Therapeutics) binding to CD3 and CD19 and AFM13 (Affimed Therapeutics) binding to CD30 and CD16A.

Antibody-drug conjugate (ADC)

In further aspect, the present invention relates to an antibody-drug conjugate (ADC) in which the antibody or antigen-binding fragment thereof is conjugated to a drug.

The anticancer-drug conjugate requires the anticancer drug to be stably attached to the antibody until the anticancer drug is delivered to the target cancer cells. The drug delivered to the target has to be released from the antibody and induce death of the target cells. To this end, the drug has to stably bind to the antibody and also possess sufficient cytotoxicity to induce death of the target cells when released from the target cells.

In one embodiment, the antibody may be conjugated to the drug via a linker. The linker is a site that connects the anti-ROR1 antibody and the drug, and allows the drug to be released from the antibody in a cleavable form under intracellular conditions, namely in the intracellular environment. Reflecting the long half-life of the antibody, the linker should be stable during systemic circulation of the antibody, and the binding of the linker to the drug should not affect the stability and pharmacokinetics of the antibody.

The linker may comprise, for example, a cleavable linker or a non-cleavable linker. For a cleavable linker, like a peptide linker, it may be cleaved by intracellular peptidase or protease enzymes, such as lysosomal or endosomal proteases, and for a non-cleavable linker, for example, a thioether linker, the antibody may be non-selectively degraded by intracellular hydrolysis and then the drug may be released.

In one embodiment, the cleavable linker may comprise a peptide linker. The peptide linker has a length of at least two amino acids. The cleavable linker may comprise a dipeptide of Val-Cit, Val-Ala, Val-Cit, or Phe-Leu, or Gly-Phe-Leu-Gly. Examples of the linker are specified in International Patent Application Publication No. WO2004/010957, which may be incorporated herein by reference.

For the antibody-drug conjugate, the antibody region of the ADC binds to the antigen of the target cancer cell to form an ADC-antigen complex, which is then internalized into the cancer cell through an endosome-lysosome pathway. As such, the intracellular release of the cytotoxic drug is controlled by the internal environment of endosomes/lysosomes.

In one embodiment, the cleavable linker is pH sensitive, meaning it may be susceptible to hydrolysis at a predetermined pH. In general, a pH sensitive linker indicates that it may be hydrolyzed under acidic conditions. Examples of an acid-labile linker that may be hydrolyzed in lysosomes may include hydrazone, semicarbazone, thiosemicarbazone, cis-aconitic amide, orthoester, acetal, ketal, and the like.

In another embodiment, the linker may be cleaved under reducing conditions, and may comprise, for example, a disulfide linker. A variety of disulfide bonds may be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), and SMPT (N-succinimidyl-oxycarbonyl-alpha-methyl-alpha-(2-pyridyl-dithio)toluene). This disulfide linker may be degraded by disulfide exchange with the thiol of intracellular glutathione.

The drug and/or drug-linker may be randomly conjugated through lysine of the antibody, or may be conjugated through cysteine exposed when the disulfide bond chain is reduced. In some cases, a linker-drug may be bound through cysteine present in a genetically engineered tag, for example, a peptide or protein. The genetically engineered tag, for example, a peptide or protein, may comprise an amino acid motif that may be recognized by, for example, isoprenoid transferase. The peptide or protein has a deletion at the carboxyl terminus of the peptide or protein, or has a covalent addition of a spacer unit to the carboxyl (C) terminus of the peptide or protein.

The peptide or protein may be directly covalently linked to the amino acid motif or may be covalently linked to the amino acid motif with a spacer unit. The amino acid spacer unit is composed of 1 to 20 amino acids, and in particular, glycine unit is preferable.

The isoprenoid transferase may be, for example, farnesyltransferase (FTase, farnesyl protein transferase) or geranylgeranyltransferase (GGTase), and FTase and GGTase I may recognize the CAAX motif of Chemical Formula 1 mentioned above, and GGTase II may recognize XXCC, XCXC, or CXX motifs (in which C is cysteine, A is an aliphatic amino acid, and X is an amino acid that determines the substrate specificity of the isoprenoid transferase).

In still another embodiment, the linker may comprise a beta-glucuronide linker that is recognized and hydrolyzed by beta-glucuronidase (β-glucuronidase), which is present in large numbers in lysosomes or overexpressed in some tumor cells. Unlike peptide linkers, this linker is highly hydrophilic and has the advantage of increasing the solubility of the antibody-drug conjugate when bound to a highly hydrophobic drug.

In this regard, the beta-glucuronide linker disclosed in International Patent Application Publication No. WO2015/182984, for example, a beta-glucuronide linker containing a self-immolative group may be used, in which the above document is incorporated herein by reference.

In some cases, the linker may be, for example, a non-cleavable linker, and the drug is released through only one step of antibody hydrolysis within the cell, producing, for example, an amino acid-linker-drug complex. This type of linker may be a thioether group or maleimidocaproyl group and may remain stable in the blood.

According to an embodiment of the present invention, the linker-drug may be randomly bound through cysteine exposed when the disulfide bond chain of the antibody is reduced or the linker-drug may be bound by introducing an antibody terminal binding peptide having the sequence GGGGGGGGCVIM.

The drug (including D in Chemical Formula (1)) is an agent that exhibits pharmacological effects and is able to bind to an antibody, and specific examples thereof may comprise a chemotherapeutic agent, a toxin, micro RNA (miRNA), siRNA, shRNA, and a radioactive isotope. The chemotherapeutic agent may be, for example, a cytotoxic agent or an immunosuppressant. Specifically, a chemotherapeutic agent capable of functioning as a microtubulin inhibitor, a mitotic inhibitor, a topoisomerase inhibitor, or a DNA intercalator may be included. Also, immunomodulatory compounds, anticancer agents, antiviral agents, antibacterial agents, antifungal agents, anthelmintic agents, or combinations thereof may be included.

For example, the drug may be, but is not limited to, at least one selected from the group consisting of maytansinoid, auristatin (including MMAE and MMAF), aminopterin, actinomycin, bleomycin, talisomycin, camptothecin, N8-acetylspermidine, 1-(2-chloroethyl)-1,2-dimethyl sulfonyl hydrazide, esperamicin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-$\alpha$, interferon-$\gamma$, tumor necrosis factor, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins derived from bacteria or animals and plants.

In some cases, the drug may comprise at least one nucleophilic group selected from the group consisting of amine, thiol, hydroxyl, hydrazide, oxime, hydrazine, thiosemicarbazone, hydrazine carboxylate, and arylhydrazide, each of which is capable of reacting to form a covalent bond with an electrophilic group on the linker and linker reagent.

In a specific embodiment according to the present invention, an ADC was produced by linking the antibody or antigen-binding fragment thereof according to the present invention to a drug, such as auristatin (MMAE), via the MC-vc-PAB linker. This ADC was confirmed to exhibit desired cytotoxicity.

Chimeric antigen receptor (CAR)

In still a further aspect, the present invention relates to a chimeric antigen receptor (CAR) comprising an extracellular domain comprising an antigen-binding site, a transmembrane domain, and an intracellular signaling domain, in which the antigen-binding site of the extracellular domain is an scFv of the antibody.

A chimeric antigen receptor (CAR) is a synthetic construct designed to induce an immune response against a target antigen and cells expressing the antigen. The CAR comprises an extracellular domain, a transmembrane domain, and an intracellular signaling domain. By introducing a gene encoding a receptor recognizing a cancer cell surface antigen specifically expressed on the surface of cancer cells into immune cells, cancer cells may be killed. Through immune cells comprising a receptor that binds to an antigen specifically expressed in cancer cells, it is possible to induce an immune response by targeting only cancer cells. The CAR comprises the scFv of the anti-ROR1 antibody according to the present invention as an antigen recognition site of the extracellular domain.

A first-generation CAR comprises an extracellular domain comprising an antigen recognition site specifically expressed in cancer cells, a transmembrane domain, and an intracellular signaling domain, in which only CD3ζ is used as the signaling domain, but is problematic in that the therapeutic effect on cancer is insignificant and the duration is short. This first-generation CAR is specified in U.S. Patent No. 6,319,494, which is incorporated herein by reference.

To improve responsiveness to immune cells, a second-generation CAR was manufactured by attaching a costimulatory domain (CD28 or CD137/4-1BB) to CD3ζ. Compared to the first-generation CAR, the second-generation CAR has a greatly increased number of CAR-comprising immune cells remaining in the body. The second-generation CAR used one costimulatory domain, while a third-generation CAR used two or more costimulatory domains. To achieve expansion and persistence of CAR-comprising immune cells *in vivo,* the costimulatory domain may be attached to 4-1BB, CD28, or OX40. The second-generation CAR is specified in U.S. Patent No. 7,741,465, 7,446,190, or 9,212,229, and the third-generation CAR is specified in U.S. Patent No. 8,822,647, all of which are incorporated herein by reference.

A fourth-generation CAR comprises an additional gene encoding cytokine such as IL-12 or IL-15, allowing additional expression of CAR-based immune protein of cytokine, and a fifth-generation CAR further comprises an interleukin receptor chain, for example IL-2Rβ, to strengthen immune cells. The fourth-generation CAR is specified in U.S. Patent No. 10,316,102, and the fifth-generation CAR is specified in U.S. Patent No. 10,336,810, all of which are incorporated herein by reference.

In one embodiment, the antigen-binding site of the extracellular domain is an scFv of an antibody. In the scFv comprising the VH and VL domains of the antibody, the VH and VL domains may be connected via a linker. The heavy chain variable region comprising at least one amino acid sequence selected from the group consisting of SEQ ID NO: 122 to SEQ ID NO: 152 and SEQ ID NO: 188 to SEQ ID NO: 193 may be connected to the light chain variable region comprising at least one amino acid sequence selected from the group consisting of SEQ ID NO: 153 to SEQ ID NO: 183 and SEQ ID NO: 194 to SEQ ID NO: 199 via a linker.

The linker may be a peptide linker and may have a length of about 10-25 aa. Examples of the linker may comprise hydrophilic amino acids such as glycine and/or serine.

The linker may include, for example, $(GS)_n$, $(GGS)_n$, $(GSGGS)_n$, or $(G_nS)_m$ (in which each of n and m is 1 to 10), but the linker may be, for example, $(G_nS)_m$ (in which each of n and m is 1 to 10). Specifically, the linker may comprise GGGGS, for example, GGGGSGGGGSGGGGS of SEQ ID NO: 200 repeated three times.

The transmembrane domain may be derived from natural or synthetic sources. When the source is natural, the domain may be derived from any membrane-binding protein or transmembrane protein. The transmembrane domain may comprise an alpha, beta, or zeta chain of T-cell receptor, CD28, CD3 epsilon, CD45, CD4, CD5, CD8, CD9, CD16, CD22, CD33, CD37, CD64, CD80, CD86, CD134, CD137, CD154, or ICOS. When synthesizing the transmembrane domain, it may comprise hydrophobic residues such as leucine and valine, or peptides including phenylalanine, tryptophan, and valine at each end. By a short oligopeptide linker, 2 to 10 amino acids in length, or a polypeptide linker, a linkage may be formed between the transmembrane domain and the cytoplasmic signaling domain of the CAR. Glycine-serine peptide may be used as the linker.

The signaling domain may induce activation of the normal effector functions of the immune cells in which the CAR is placed. For example, the signaling domain may induce cytolytic activation or helper activation through secretion of cytokines. The signaling domain may comprise a truncated fragment of an intracellular signaling domain sufficient to transduce an effector function signal.

The signaling domain may comprise the cytoplasm of the T cell receptor (TCR) and co-receptor that act cooperatively to initiate signaling following antigen receptor engagement.

It is also known that signals generated through the TCR alone are insufficient for full activation of T cells and that costimulatory signals are required. Therefore, T cell activation may involve initiating antigen-dependent primary activation via the TCR and acting in an antigen-dependent manner to provide secondary or costimulatory signals. Primary cytoplasmic signaling sequences regulate primary activation of the TCR complex in a stimulatory or an inhibitory manner. Primary cytoplasmic signaling sequences that act in a stimulatory manner may contain signaling motifs known as immunoreceptor tyrosine-based activation motifs or ITAMs. Examples of ITAMs containing primary cytoplasmic signaling sequences may comprise TCR zeta, FcR gamma, FcR beta, CD3 gamma, CD3 delta, CD3 epsilon, CD5, CD22, CD79a, CD79b, and CD66d.

In some cases, the cytoplasmic domain of the CAR may comprise a CD3 zeta chain portion and a costimulatory signaling region. The costimulatory signaling region may be a portion of the CAR that comprises the intracellular domain of the costimulatory molecule. Examples thereof may comprise ligands and the like that specifically bind to CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and CD83. The cytoplasmic signaling sequences in the cytoplasmic signaling portion of the CAR may be linked via a peptide linker comprising 2 to 10 amino acids, for example, glycine-serine.

In yet a further aspect, the present invention relates to an immune cell into which the chimeric antigen receptor (CAR) is introduced.

The immune cell is capable of inducing immunity to obtain the desired cancer treatment effect, and for example, may be selected from the group consisting of T cells, NK cells, cytokine induced killer cells (CIKs), activated cytotoxic T lymphocytes (CTLs), macrophages, tumor-infiltrating lymphocytes (TILs), and dendritic cells, but is not limited thereto.

An antibody other than the antibody described above may be an antibody or antigen-binding fragment thereof targeting at least one selected from the group consisting of, for example, PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO.

Therapeutic composition

In still yet a further aspect, the present invention relates to a composition for preventing or treating cancer, comprising the antibody or antigen-binding fragment thereof, the bispecific or multispecific antibody comprising the antibody or antigen-binding fragment thereof, the antibody-drug conjugate comprising the antibody or antigen-binding fragment thereof, the chimeric antigen receptor comprising the antibody or antigen-binding fragment thereof, or the immune cell comprising the chimeric antigen receptor.

For example, the present invention may provide a pharmaceutical composition for preventing or treating cancer, comprising (a) a pharmaceutically effective amount of the anti-ROR1 antibody or antigen-binding fragment thereof according to the present invention, the bispecific or multispecific antibody comprising the antibody or antigen-binding fragment thereof, the antibody-drug conjugate comprising the antibody or antigen-binding fragment thereof, the chimeric antigen receptor comprising the antibody or antigen-binding fragment thereof, or the immune cell comprising the chimeric antigen receptor; and (b) a pharmaceutically acceptable carrier. In addition, the present invention may provide a method of preventing or treating cancer, comprising administering, to a cancer patient, the anti-ROR1 antibody or antigen-binding fragment thereof according to the present invention, the bispecific or multispecific antibody comprising the antibody or antigen-binding fragment thereof, the antibody-drug conjugate comprising the antibody or antigen-binding fragment thereof, the chimeric antigen receptor comprising the antibody or antigen-binding fragment thereof, or the immune cell comprising the chimeric antigen receptor.

The term "prevention" refers to any action that inhibits the growth of cancer or delays the progression thereof by administering the composition according to the present invention, and the term "treatment" refers to inhibition of cancer development, alleviation of tumor, or removal of cancer.

Examples of such cancer include Hodgkin's lymphoma, non-Hodgkin's lymphoma (e.g., B cell lymphoma, diffuse large B cell lymphoma, follicular lymphoma, chronic lymphocytic leukemia, mantle cell lymphoma, marginal zone B cell lymphoma, Burkitt's lymphoma, lymphoplasmacytic lymphoma, and hairy cell leukemia), acute myeloid leukemia, chronic myeloid leukemia, myelodysplastic syndrome, multiple myeloma, and acute lymphocytic leukemia.

Examples of the cancer include ovarian cancer, rectal cancer, stomach cancer, testicular cancer, anal cancer, uterine cancer, colon cancer, rectal cancer, renal cell carcinoma, liver cancer, non-small cell lung carcinoma, small intestine cancer, esophageal cancer, melanoma, Kaposi's sarcoma, endocrine cancer, thyroid cancer, parathyroid cancer, adrenal cancer, bone cancer, pancreatic cancer, skin cancer, head and neck cancer, skin or intraocular malignant melanoma, uterine cancer, brain stem glioma, pituitary adenocarcinoma, epidermoid cancer, cervical squamous cell carcinoma, fallopian tube carcinoma, endometrial carcinoma, vaginal carcinoma, soft tissue sarcoma, urethral cancer, vulvar carcinoma, penile cancer, bladder cancer, kidney or ureter cancer, renal pelvic carcinoma, spinal tumor, neoplasm of the central nervous system (CNS), primary CNS lymphoma, angiogenic tumor, metastatic lesions thereof, or combinations thereof.

The cancer may be, for example, glioblastoma, lung cancer, bladder cancer, oral cancer, head and neck squamous cell cancer, gallbladder cancer, or cervical cancer.

The pharmaceutically acceptable carrier comprised in the composition of the present invention comprises those commonly used in formulation, and examples thereof include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, water, syrup, methyl cellulose, methylhydroxybenzoate, propylhydroxybenzoate, talc, magnesium stearate, and mineral oil. The composition of the present invention may further comprise a lubricant, a wetting agent, a sweetening agent, a flavoring agent, an emulsifying agent, a suspending agent, a preservative, and the like, in addition to the above components.

The pharmaceutical composition of the present invention may be administered orally or parenterally, and parenteral administration may include intravenous injection, subcutaneous injection, intramuscular injection, intraperitoneal injection, endothelial administration, topical administration, intranasal administration, intrapulmonary administration, and intrarectal administration.

When administered orally, the protein or peptide is digestible and therefore oral compositions have to be formulated to coat the active agent or to protect the same from degradation in the stomach. Also, the pharmaceutical composition may be administered by any device that allows the active material to move to target cells.

A suitable dosage of the composition according to the present invention may vary depending on factors such as formulation method, administration mode, patient's age, body weight, gender, morbidity, food, administration time, administration route, excretion rate, and reaction sensitivity, and an effective dosage for the desired treatment or prevention may be easily determined and prescribed by a skilled doctor. For example, a daily dosage of the pharmaceutical composition of the present invention is 0.0001-100 mg/kg. As used herein, the term "pharmaceutically effective amount" refers to an amount sufficient to prevent or treat cancer or autoimmune disease.

The pharmaceutical composition of the present invention may be prepared in a unit dosage form or may be prepared by being placed in a multidose container, by formulating the same using a pharmaceutically acceptable carrier and/or excipient according to a method that may be easily performed by those skilled in the art. As such, the formulation may be in the form of a solution, suspension or emulsion in oil or aqueous medium, or may be in the form of an extract, powder, suppository, dust, granule, tablet, or capsule, and may additionally comprise a dispersant or stabilizer.

Treatment method

In even still a further aspect, the present invention relates to a composition for treating cancer, comprising the antibody or antigen-binding fragment thereof, the bispecific or multispecific antibody comprising the antibody or antigen-binding fragment thereof, the antibody-drug conjugate, the chimeric antigen receptor, or the immune cell comprising the chimeric antigen receptor.

In addition, the present invention relates to a method of treating cancer, comprising administering the antibody or antigen-binding fragment thereof, the bispecific or multispecific antibody comprising the antibody or antigen-binding fragment thereof, the antibody-drug conjugate, the chimeric antigen receptor, or the immune cell comprising the chimeric antigen receptor.

In even yet a further aspect, the present invention relates to the use of the antibody or antigen-binding fragment thereof, the bispecific or multispecific antibody comprising the antibody or antigen-binding fragment thereof, the antibody-drug conjugate, the chimeric antigen receptor, or the immune cell comprising the chimeric antigen receptor for the prevention or treatment of cancer.

In even still yet a further aspect, the present invention relates to the use of the antibody or antigen-binding fragment thereof, the bispecific or multispecific antibody comprising the antibody or antigen-binding fragment thereof, the antibody-drug conjugate, the chimeric antigen receptor, or the immune cell comprising the chimeric antigen receptor for the manufacture of a medicament for preventing or treating cancer.

Combination therapy

The present invention relates to a composition for combination therapy comprising an immune cell and a drug other than the anti-ROR1 antibody.

In one embodiment, the drug other than the anti-ROR1 antibody may comprise a chemotherapeutic agent or an antibody other than the anti-ROR1 antibody.

The drug may be at least one selected from the group consisting of maytansinoid, auristatin (including MMAE and MMAF), aminopterin, actinomycin, bleomycin, talisomycin, camptothecin, N8-acetylspermidine, 1-(2-chloroethyl)-1,2-dimethyl sulfonyl hydrazide, esperamicin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-$\alpha$, interferon-$\gamma$, tumor necrosis factor, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins derived from bacteria or animals and plants.

In one embodiment, the antibody other than the anti-ROR1 antibody may be an antibody or antigen-binding fragment thereof targeting at least one selected from the group consisting of, for example, PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO.

The present invention relates to a composition for combination therapy comprising the antibody or antigen-binding fragment thereof and at least one selected from the group consisting of:

(i) an immune cell;
(ii) an immune cell comprising a chimeric antigen receptor (CAR) comprising an scFv of an antibody other than the anti-ROR1 antibody as an extracellular domain; and
(iii) an immune checkpoint inhibitor.

In addition, the present invention relates to a composition for combination therapy comprising the immune cell-engaging bispecific or multispecific antibody and at least one selected from the group consisting of:

(i) an immune cell;
(ii) an immune cell comprising a chimeric antigen receptor (CAR) comprising an scFv fragment of an antibody other than the anti-ROR1 antibody as an extracellular domain; and
(iii) an immune checkpoint inhibitor.

The immune cell is capable of inducing immunotherapy, for example, immunity to obtain the desired cancer treatment effect, and may be selected from the group consisting of, for example, T cells, NK cells, cytokine induced killer cells (CIKs), activated cytotoxic T lymphocytes (CTLs), macrophages, tumor-infiltrating lymphocytes (TILs), and dendritic cells, but is not limited thereto.

The antibody other than the anti-ROR1 antibody is an antibody targeting a target other than ROR1, and may be an antibody or antigen-binding fragment thereof that binds to, for example, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, or MARCO, but is not limited thereto.

The immune checkpoint inhibitor is an agent that may induce T cell activation by blocking T cell inhibitory signals at the site where antigen presenting cells (APC) and immune cells, such as T cells, meet. The immune checkpoint inhibitor may be a drug targeting, for example, PD-1, PD-L1, BTLA, CTLA-4, VISTA, LAG3, TIM3, CD137 (4-1BB), CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, or CD200R, but is not limited thereto.

The first and second components to be co-administered may be administered simultaneously. Alternatively, the first and second components to be co-administered may be administered separately at predetermined time intervals. Among the components to be co-administered, the second component may be administered separately before or after administration of the first component.

A better understanding of the present invention may be obtained through the following examples. These examples are merely set forth to illustrate the present invention, and are not to be construed as limiting the scope of the present invention, as will be apparent to those of ordinary skill in the art.

**Example 1: Preparation of phage library (scFv)**

To recover, in phage form, phagemid vectors having human-derived synthetic scFv library genes constructed conventionally, six sub-library samples ((1) Bai, Xuelian & Kim, Jihye & Kang, Seungmin & Kim, Wankyu & Shim, Hyunbo. (2015). A Novel Human scFv Library with Non-Combinatorial Synthetic CDR Diversity. PloS one. 10. e0141045. 10.1371/journal.pone.0141045., (2) Yang, Hye & Kang, Kyung & TCW, Julia & Shim, Hyunbo. (2009). Construction of a large synthetic human scFv library with six diversified CDRs and high functional diversity. Molecules and cells. 27. 225-35. 10.1007/s10059-009-0028-9.) were each cultured for 2 hours in 400 ml of culture medium (SB/ampicillin/2% glucose). When the absorbance reached 0.5-0.7 at $OD_{600}$, centrifugation was performed at 5,000 g for 20 minutes to remove the supernatant, and then $10^{12}$ pfu (plaque forming unit) of helper phage (VCSM13) was added to 400 ml of secondary culture medium (SB/ampicillin) followed by re-culture for 1 hour. Thereafter, kanamycin antibiotic (an antibiotic gene introduced into the helper phage) was added at a concentration of 70 $\mu$g/ml followed by culture with shaking at 30°C and 250 rpm for 16 hours to enable the phage library to be produced outside host cells. Thereafter, PEG 8000 (polyethylene glycol 8000) and NaCl were added to the supernatant resulting from centrifugation of the culture, and the recombinant phage was precipitated with stirring at 4°C for 2 hours. The phage library was recovered by performing centrifugation at 10,000 g and 4°C for 30 minutes, suspending the precipitated pellets by addition of PBS, performing centrifugation at 15,000 g and 4°C for 30 minutes, and adding PBS to the precipitated phage pellets. The concentration of the amplified sub-library was calculated as the number of colonies generated by diluting the recovered phage, infecting TG1 cells therewith, and culturing the same on LB/ampicillin solid culture medium.

**Example 2: Selection of anti-ROR1 specific antibody (scFv) using biopanning**

Panning was performed to select human antibodies specifically binding to ROR1. Biopanning was performed using ROR1-Fc protein, ROR1-His protein, and patient-derived cells as follows. Human ROR1 Fc protein (R&D Systems, 9490-RO, Recombinant Human ROR1 Fc Chimera Protein, CF) and human ROR1 His protein (Sino Biological, 13968-H08H, ROR1 Protein, Human, Recombinant (ECD, His Tag)) were used as recombinant antigens, and LC-074T, which overexpresses ROR1 possessed by Aimedbio, was used as the patient-derived cells.

Antigen-immobilized biopanning: A 96-well plate was coated at 4°C for 16 hours with 5 to 10 μg/ml of human ROR1 Fc protein and negative Fc protein and then blocked using 3% skim milk. After emptying the plate, the library was added to the plate coated with (about $2.0 \times 10^{13}$ pfu) negative Fc protein and reacted at room temperature for 30 minutes. This is a step to prevent non-specific binding other than to ROR1 by removing phages that bind to proteins other than human ROR1 from the antibody phage library. Phages unbound to the negative Fc protein were recovered and allowed to bind to the plate coated with human ROR1 Fc for 1 hour. After washing five to nine times with PBST (phosphate buffered saline-0.05% Tween 20) solution to remove non-specific binding, phage antibodies specific to human ROR1 were recovered using IgG elution buffer (Thermo Scientific, 21028, Pierce™ IgG Elution Buffer, pH 2.0). A total of three rounds was conducted, and the results of antigen-immobilized biopanning are shown in Table 1 below.

Bead-based biopanning: Bead panning was repeated by attaching magnetic beads to biotinylated human ROR1 protein. Streptavidin-conjugated magnetic beads (Invitrogen, 11206D, Dynabeads™ M-280 Streptavidin) were used, and human ROR1 protein was biotinylated using a biotinylation kit (Abcam, ab201795, Biotin Conjugation Kit (Fast, Type A) - Lightning-Link®). After washing 100 μl of Dynabeads™ M-280 streptavidin beads with PBST (phosphate buffered saline-0.1% Tween 20) solution and PBS solution using a magnetic bead separator, 50-100 nM biotinylated human ROR1 protein was reacted at room temperature for 30 minutes. Thereafter, the library was collected to about $1.0 \times 10^{13}$ pfu and then blocked using 3% skim milk. The blocked library and the pre-reacted biotinylated human ROR1-bead conjugate were mixed and reacted at room temperature for 2 hours. Thereafter, the library bound to human ROR1 beads was recovered using a magnetic bead separator, and washed 7-12 times with PBST (phosphate buffered saline-0.1% Tween 20) solution and PBS solution, followed by elution of phage antibodies bound to biotinylated human ROR1-beads using IgG elution buffer (Thermo Scientific, 21028, Pierce™ IgG Elution Buffer, pH 2.0). A total of three rounds was conducted, and the results of bead-based biopanning are shown in Table 2 below.

Biopanning using patient-derived cells: Jurkat cells were treated with the phage antibodies obtained from antigen-immobilized biopanning and bead-based biopanning and allowed to bind at 4°C for 1 hour, and the supernatant unbound to ROR1-overexpressed patient-derived cells was recovered. This is a step to prevent non-specific binding other than to ROR1 by removing phages that bind to cell membrane proteins other than ROR1 from the phage antibody. LC-074T ($1.5 \times 10^6$) as ROR1 patient-derived cells were treated with the recovered supernatant and allowed to bind at 4°C for 1 hour. Thereafter, to remove phages unbound to the patient-derived cells, they were transferred to a 15 ml conical tube and then centrifuged at 1,000 g for 3 minutes to separate the cells, followed by washing three to five times with 3 ml of cold PBS. Thereafter, the phages on the cell surface were separated from the cell surface by leaving on ice for 10 minutes using IgG elution buffer (Thermo Scientific, 21028, Pierce™ IgG Elution Buffer, pH 2.0). After centrifugation at 1,000 g for 3 minutes, 50 μl of lysis buffer was added to the cells, and the cells were lysed by leaving on ice for 10 minutes. The cells were centrifuged at 12,000 rpm for 5 minutes to separate cell debris, after which the supernatant with phage particles in the cells and the phages isolated from the cell surface were added to culture medium (SB) containing pre-grown TG1, followed by culture at 37°C and 120 rpm for 1 hour to infect TG1 cells with phage particles. Thereafter, the cells were plated on LB/ampicillin solid medium, the remaining solution was centrifuged at 4,000 rpm for 10 minutes, and the precipitated TG1 was plated on 15 cm LB/ampicillin solid medium, followed by culture. Then, 5 ml of SB culture medium (50% glycerol) was added and thus colonies were recovered and stored (-80°C). Then, in order to repeat panning rounds, 50 μl of the phage solution stored in the previous round was taken and phage particle amplification was performed. After culture, the phage particles recovered by adding helper phage were separated through PEG precipitation and were used in the same manner for the next round of panning. A total of two rounds was conducted, and the results of biopanning using patient-derived cells are shown in Table 3 below. Three rounds were conducted using the phage antibodies obtained from the patient-derived cells as recombinant proteins, and the results thereof are shown in Table 4 below. It was confirmed that, as the rounds continued, the proportion of phage particles after panning compared to before panning increased.

[Table 1]

| Round | Input (CFU/ml) | Output (CFU/ml) | Recovery rate (Output/Input) |
|---|---|---|---|
| 1 | 2.33E+13 | 1.67E+06 | 7.17E-08 |
| 2 | 1.81E+13 | 7.67E+06 | 4.24E-07 |
| 3 | 1.73E+13 | 3.59E+07 | 1.08E-06 |

[Table 2]

| Round | Input (CFU/ml) | Output (CFU/ml) | Recovery rate (Output/Input) |
|---|---|---|---|
| 1 | 4.13E+12 | 1.65E+06 | 3.98E-07 |
| 2 | 8.70E+12 | 8.19E+05 | 9.41E-08 |
| 3 | 1.65E+12 | 6.67E+06 | 4.04E-06 |

[Table 3]

| Round | Input (CFU/ml) | Output (CFU/ml) | Recovery rate (Output/Input) |
|---|---|---|---|
| 1 | 5.36E+12 | 5.15E+06 | 9.61E-07 |
| 2 | 3.06E+12 | 3.73E+06 | 1.22E-06 |

[Table 4]

| Round | Input (CFU/ml) | Output (CFU/ml) | Recovery rate (Output/Input) |
|---|---|---|---|
| 1 | 1.15E+13 | 3.59E+05 | 3.12E-08 |
| 2 | 1.37E+13 | 5.26E+07 | 3.84E-06 |
| 3 | 1.32E+12 | 5.19E+07 | 3.93E-05 |

**Example 3: Affinity ELISA screening and sequence analysis of anti-ROR1 specific antibody (scFv)**

ScFv screening was performed to select monoclonal antibodies that specifically bind to ROR1 from the phages recovered through the final round of panning. Colonies from the final round of panning were taken, inoculated into a 96-well plate containing 200 μl of SB/ampicillin culture medium, and then cultured at 37°C for 2-3 hours. Thereafter, to induce scFv-pIII protein expression, each well was treated with IPTG (isopropyl β-D-1-thiogalactopyranoside) at a final concentration of 1 mM and cultured overnight at 30°C. The cultured plate was centrifuged at 3,000 rpm for 15 minutes to remove the supernatant, after which 40 μl of TES (50 mM Tris, 1 mM EDTA, 20% sucrose, pH 8.0) solution was added to each well to recover phage particles, and the cells were lysed at room temperature for 30 minutes. Thereafter, the cells were treated with 60 μl of 0.2X TES solution and lysed by leaving at 4°C for 2 hours, then the plate was centrifuged at 3,000 rpm for 15 minutes to collect the supernatant.

The supernatant was added to each well in a 96-well plate coated with human ROR1, mouse ROR1, and negative Fc protein, followed by binding at room temperature for 2 hours and then washing four times with PBST and distilled water. After binding at room temperature for 1 hour using an HRP-conjugated anti-HA antibody capable of binding to the HA tag, washing was performed six times using PBST and distilled water. After color development by adding TMB substrate solution (Thermo Scientific, 34029, 1-Step™ Ultra TMB-ELISA Substrate Solution), the color reaction was stopped with stop solution (Invitrogen, SS04, ELISA Stop Solution), and the absorbance was measured at OD 450 nm. 30 antibody clones binding to human ROR1 or mouse ROR1 were selected through ELISA, and the CDR sequences of each antibody are shown in Table 5 below, and the amino acid sequences of the heavy and light chain variable regions are shown in Table 6 below.

[Table 5]

| | CDR H1 | | CDR H2 | | CDR H3 | |
|---|---|---|---|---|---|---|
| | Sequence | SEQ ID NO: | Sequence | SEQ ID NO: | Sequence | SEQ ID NO: |
| P015001 | NYDMS | 1 | GIYSGNSNTYYADSVKG | 17 | DLQYTEWAGFDY | 42 |
| P015002 | DYSMS | 2 | GISHNGGSTYYADSVKG | 18 | DVSRWSKTRWSYDNAMDV | 43 |
| P015003 | SYAMS | 3 | GISPGGSSKYYADSVKG | 19 | RGPHRAIDTAFDY | 44 |
| P015004 | DYAMA | 4 | IIYPGDSETIYSPSFEG | 20 | RDTYWEGYFDL | 45 |
| P015005 | DYSMS | 2 | GISHNGGSTYYADSVKG | 18 | DVSRWSKTRWSYDNAMDV | 43 |
| P015006 | NYSMS | 5 | AISHGSGSIYYADSVKG | 21 | KPISAWSHIRMSYAYGMDV | 46 |
| P015007 | GYSMS | 6 | GISYGGSSIYADSVKG | 22 | KPVRRWARFRLSYDDAMDV | 47 |
| P015009 | NYSMS | 5 | GIYSGNSNTYYADSVKG | 17 | DSRATHHRWHYYDNAMDV | 48 |
| P015010 | NYAMS | 7 | GISPNGSSIYYADSVKG | 23 | KAARLSPSRNYSYAYGMDV | 49 |
| P015011 | NYAMS | 7 | GISPDGGSIYYADSVKG | 24 | KAARLSPSMNYSYAYGMDV | 50 |
| P015012 | GYSMS | 6 | GISYGGSSISYADSVKG | 25 | KPVRRWARFRLSYDDAMDV | 47 |
| P015013 | SYDMS | 8 | WISHGGGSIYYADSVKG | 26 | DLSNRLFTGFDY | 51 |
| P015014 | SYSMS | 9 | ISPGDSNKYYADSVKG | 27 | TFPYFDY | 52 |
| P015015 | NYAMS | 7 | GISPGGGSIYYADSVKG | 28 | KAARLSPSMNYSYAYGMDV | 50 |

(continued)

| | CDR H1 | | CDR H2 | | CDR H3 | |
|---|---|---|---|---|---|---|
| | Sequence | SEQ ID NO: | Sequence | SEQ ID NO: | Sequence | SEQ ID NO: |
| P015016 | DYAMS | 10 | AISHGGSSKYYADSV KG | 29 | RVSLDFDY | 53 |
| P015017 | GYAMS | 11 | VISPGSGSKYYADSV RG | 30 | RYPLLRYAGALYSAY AMDV | 54 |
| P015018 | SYAKH | 12 | IYTSGSTNYNPSLKS | 31 | DTWWEGYFDL | 55 |
| P015020 | GYYMS | 13 | VISHDGSSTYYADSV KG | 32 | VKYIFDY | 56 |
| P015021 | SYAMS | 3 | WISPGGGNIYYADSV KG | 33 | RSQSGFDY | 57 |
| P015022 | NYDMS | 1 | SISSNGGSTYYADSV KG | 34 | CLLQFDY | 58 |
| P015023 | GYDMS | 14 | GISHDGRSKYYADSV KG | 35 | KDVPFKMRFIFDY | 59 |
| P015024 | DYYMS | 15 | LISPGSDSKYYADSV KG | 36 | KIEPDFDY | 60 |
| P015028 | DYAMS | 10 | AISYDNGNTYYADSV KG | 37 | VNHRFDY | 61 |
| P015038 | NYAMS | 7 | GISPNGSSIYYADSV KG | 23 | KAARLSPSRNYSYAY GMDV | 49 |
| P015039 | DYAMS | 10 | WISPGGGNTYYADSV KG | 38 | KGALMCLSATCSSDY GMDV | 62 |
| P015040 | NYAMS | 7 | GISPNGSSIYYADSV KG | 23 | KAARLSPSRNYSYAY GMDV | 49 |
| P015041 | NYAMS | 7 | GISPNGSSIYYADSV KG | 23 | KAARLSPSRNYSYAY GMDV | 49 |
| P015042 | DYAMS | 10 | VISSDGGSIYYADSV KG | 39 | DRSRDMEVDFDY | 63 |
| P015043 | NYDMS | 1 | WISPGGSSIYYADSV KG | 40 | TLTRFDY | 64 |

(continued)

| | CDR H1 | | CDR H2 | | CDR H3 | |
|---|---|---|---|---|---|---|
| | Sequence | SEQ ID NO: | Sequence | SEQ ID NO: | Sequence | SEQ ID NO: |
| P015044 | NYAMH | 16 | SIIYPDDDTRYNPSF RG | 41 | RDLLYAGDY | 65 |

| | CDR L1 | | CDR L2 | | CDR L3 | |
|---|---|---|---|---|---|---|
| | Sequence | SEQ ID NO: | Sequence | SEQ ID NO: | Sequence | SEQ ID NO: |
| P015001 | SGSSSNIGSNDVT | 66 | DDSKRPS | 87 | AAWDDSLSGYV | 103 |
| P015002 | TGSSSNIGSNYVS | 67 | SDSNRPS | 88 | ATWDASLSAYV | 104 |
| P015003 | TGSSSNIGSNYVS | 67 | SDSNRPS | 88 | ATWDASLSAYV | 104 |
| P015004 | RASQGISNYLA | 68 | DASNLES | 89 | QQYANLPYT | 105 |
| P015005 | SGSSSNIGSNYVN | 69 | SDSHRPS | 90 | GSWDSSLNAYV | 106 |
| P015006 | TGSSSNIGSNYVS | 67 | SDSNRPS | 88 | ATWDASLSAYV | 104 |
| P015007 | SGSSSNIGNNAVN | 70 | ADSHRPS | 91 | GTWDSSLNGYV | 107 |
| P015009 | TGSSSNIGSNYVS | 67 | SDSNRPS | 88 | GTWDSSLSGYV | 108 |
| P015010 | TGSSSNIGNNNVT | 71 | SDSQRPS | 92 | GTWDYSLNGYV | 109 |
| P015011 | TGSSSNIGNNYVS | 72 | SDSHRPS | 90 | GTWDYSLNGYV | 109 |
| P015012 | SGSSSNIGNNAVN | 70 | ANSHRPS | 93 | GTWDSSLSGYV | 108 |
| P015013 | SGSSSNIGSNSVY | 73 | SDSHRPS | 90 | GTWDYSLNAYV | 110 |
| P015014 | SSSSSNIGSNYVN | 74 | DNSQRPS | 94 | GSWDDSLSGYV | 111 |
| P015015 | TGSSSNIGNNYVT | 75 | SDSQRPS | 92 | GTWDDSLNGYV | 112 |
| P015016 | SGSSSNIGSNTFN | 76 | ADSHRPS | 91 | AAWDASLSAYV | 113 |
| P015017 | SGSSSNIGSNYVS | 77 | ANSHRPS | 93 | GAWDSSLNGYV | 114 |
| P015018 | RASQSVSTYLA | 78 | DASSLES | 95 | QQSYNLPHT | 115 |
| P015020 | TGSSSNIGNNTVN | 79 | ADSNRPS | 96 | DTWDSSLNAYV | 116 |
| P015021 | SGSSSNIGNNSVT | 80 | YDSHRPS | 97 | GAWDSSLNGYV | 114 |
| P015022 | TGSSSNIGSNAVN | 81 | YDNQRPS | 98 | AAWDDSLSGYV | 103 |
| P015023 | TGSSSNIGSNTVY | 82 | ADSQRPS | 99 | GSWDSSLNAYV | 106 |
| P015024 | SGSSSNIGSNTFN | 76 | ADSHRPS | 91 | ATWDYSLSAYV | 117 |
| P015028 | SGSSSNIGSNTFN | 76 | ANSHRPS | 93 | ASWDASLSGYV | 118 |
| P015038 | TGSSSNIGNNNVT | 71 | SDSQRPS | 92 | GTWDYSLNGYV | 109 |
| P015039 | TGSSSNIGNNAVS | 83 | SDNQRPS | 100 | GTWDYSLNGYV | 109 |
| P015040 | TGSSSNIGNNNVT | 71 | SDSQRPS | 92 | GTWDYSLNGYV | 109 |
| P015041 | TGSSSNIGNNNVT | 71 | SDSQRPS | 92 | GTWEYSLNGYV | 119 |
| P015042 | SGSSSNIGSNYVT | 84 | DSSQRPS | 101 | GTWDYSLSGYV | 120 |
| P015042v1 | SGSSSNIGSNYVT | 84 | DSSQRPS | 101 | GTWDYSLNGYV | 109 |
| P015043 | TGSSSNIGNNNVS | 85 | YDSHRPS | 97 | GTWDSSLSGYV | 108 |

(continued)

| | CDR L1 | | CDR L2 | | CDR L3 | |
|---|---|---|---|---|---|---|
| | Sequence | SEQ ID NO: | Sequence | SEQ ID NO: | Sequence | SEQ ID NO: |
| P015044 | SCGGNNIESKAVH | 86 | ANNKRPS | 102 | QSYDSNKVVFG | 121 |

[Table 6]

| | VH Sequence | SEQ ID NO: |
|---|---|---|
| P015001 | EVQLLESGGGLAQPGGSLRLSCAASGFTFSNYDMSWVRQAPGKGPEWVSGIYSGNS NTYYADSVKGRFTISRDNSKNTLYLQMNSLRAEDTAVYYCARDLQYTEWAGFDYWG QGTLVTVSS | 122 |
| P015002 | EVQLLESGGGLVQPGGSLRLSCAASGFTFSDYSMSWVRQAPGKGLEWVSGISHNGG STYYADSVKGRFTISRDNSKNTLYLQMSSLRAEDTAVYYCARDVSRWSKTRWSYDN AMDVWGQGTLVTVSS | 123 |
| P015003 | EVQLLESGGGLVQPGGSLRLSCAASGFTFSSYAMSWVRQAPGKGLEWVSGISPGGS SKYYADSVKGRFTISRDNSKNTLYLQMNSLRAEDTAVYYCARGPHRAIDTAFDYWG QGTLVTVSS | 124 |
| P015004 | EVQLLESGGGLVQPGGSLRLSCAASGFTFSDYAMAWVRQAPGKGLEWVSIIYPGDS ETIYSPSFEGRFTISRDNSKNTLYLQMNSLRAEDTAVYYCARDTYWEGYFDLWGQG TLVTVSS | 125 |
| P015005 | EVQLLESGGGLVQPGGSLRLSCAASGFTFSDYSMSWVRQAPGKGLEWVSGISHNGG STYYADSVKGRFTISRDNSKNTLYLQMSSLRAEDTAVYYCARDVSRWSKTRWSYDN AMDVWGQGTLVTVSS | 126 |
| P015006 | EVQLLESGGGLVQPGGSLRLSCAASGFTFSNYSMSWVRQAPGKGLEWVSAISHGSG SIYYADSVKGRFTISRDNSKNTLYLQMNSLRAEDTAVYYCAKPISAWSHIRMSYAY GMDVWGQGTLVTVSS | 127 |
| P015007 | EVQLLESGGGLVQPGGSLRLSCAASGFTFSGYSMSWVRQAPGKGLEWVSGISYGGS SISYADSVKGRFTISRDNSKNTLYLQMNSLRAEDTAVYYCAKPVRRWARFRLSYDD AMDVWGQGTLVTVSS | 128 |
| P015009 | EVQLLESGGGLVQPGGSLRLSCAASGFTFSNYSMSWVRQAPGKGLEWVSGIYSGNS NTYYADSVKGRFTISRDNSKNTLYLQMNSLRAEDTAVYYCAKDSRATHHRWHYYDN AMDVWGQGTLVTVSS | 129 |

(continued)

| | | VH Sequence | SEQ ID NO: |
|---|---|---|---|
| | P015010 | EVQLLESGGGLVQPGGSLRLSCAASGFTFSNYAMSWVRQAPGKGLEWVSGISPNGS SIYYADSVKGRFTISRDNSKNTLYLQMNSLRAEDTAVYYCAKAARLSPSRNYSYAY GMDVWGQGTLVTVSS | 130 |
| | P015011 | EVQLLESGGGLVQPGGSLRLSCAASGFTFSNYAMSWVRQAPGKGLEWVSGISPDGG SIYYADSVKGRFTISRDNSKNTLYLQMNSLRAEDTAVYYCAKAARLSPSMNYSYAY GMDVWGQGTLVTVSS | 131 |
| | P015012 | EVQLLESGGGLVQPGGSLRLSCAASGFTFSGYSMSWVRQAPGKGLEWVSGISYGGS SISYADSVKGRFTISRDNSKNTLYLQMNSLRAEDTAVYYCAKPVRRWARFRLSYDD AMDVWGQGTLVTVSS | 132 |
| | P015013 | EVQLLESGGGLVQPGGSLRLSCAASGFTFSSYDMSWVRQVPGKGLEWVSWISHGGG SIYYADSVKGRFTISRDNSKNTLYLQMNSLRAEDTAVYYCARDLSNRLFTGFDYWG QGTLVAVSS | 133 |
| | P015014 | EVQLLESGGGLVQPGGSLRLSCAVSGFTFSSYSMSWVRQAPGKGLEWVSAISPGDS NKYYADSVKGRFTISRDNSKNTLYLQINSLRAEDTAVYYCARTFPYFDYWGQGTLV TVSS | 134 |
| | P015015 | EVQLLESGGGLVQPGGSLRLSCAASGFTFSNYAMSWVRQAPGKGLEWVSGISPGGG SIYYADSVKGRFTISRDNSKNTLYLQMNSLRAEDTAVYYCAKAARLSPSMNYSYAY GMDVWGQGTLVTVSS | 135 |
| | P015016 | EVQLLESGGGLVQTGGSLRLSCAASGFTFSDYAMSWVRQAPGKGLEWVSAISHGGS SKYYADSVKGRFTISRDNSKNTLYLQMNSLRAEDTAVYYCARVSLDFDYWGQGTLV AVSS | 136 |
| | P015017 | EVQLLESGGGLVQPGGSLRLSCAASGFTFSGYAMSWVRQAPGKGLEWVSVISPGSG SKYYADSVRGRFTVSRDNSKNTLYLQMNSLRAEDTAVYYCARYPLLRYAGALYSAY AMDVWGQGTLVTVSS | 137 |
| | P015018 | EVQLLESGGGLVQPDGSMRLICAASGFTFSSYAKHWVRQAPGKGLEWSEIYTSGS TNYNPSLKSRFTISRDNSKNTLYLQMNSLRAEDTAVYYCARDTWWEGYFDLWGQGT LVTVSS | 138 |
| | P015020 | EVQLLESGGGLVQPGGSLRLSCAASGFTFSGYYMSWVRQAPGKGLEWSVISHDGS STYYADSVKGRFTISRDNSKNTLYLQMNSLRAEDTAVYYCAKVKYIFDYWGQGTLV TVSS | 139 |

(continued)

| | VH Sequence | SEQ ID NO: |
|---|---|---|
| P015021 | EVQLLESGGGLVQPGGSLRLSCAASGFTFSSYAMSWVRQAPGKGLEWVSWISPGGG NIYYADSVKGRFTISRDNSKNTLYLQMNSLRAEDTAVYYCARSQSGFDYWGQGTLV TVSS | 140 |
| P015022 | EVQLLESGGGLVQPGGSLRLSCAASGFTFSNYDMSWVRQAPGKGLEWVSSISSNGG STYYADSVKGRFTISRDNSKNTLYLQMNSLRAEDTAVYYCAKCLLQFDYWGQGTLV TVSS | 141 |
| P015023 | EVQLLESGGGLVQPGGSLRLSCAASGFTFSGYDMSWVRQAPGKGLEWVSGISHDGR SKYYADSVKGRFTVSRDNSKNTLYLQMNSLRAEDTAVYYCAKDVPFKMRFIFDYWG QGTLVTVSS | 142 |
| P015024 | EVQLLESGGGLVQPGGSLRLSCAASGFTFSDYYMSWVRQAPGKGLEWVSLISPGSD SKYYADSVKGRFTISRDNSKNTLYLQMNSLRAEDTAVYYCAKIEPDFDYWGQGTLV TVSS | 143 |
| P015028 | EVQLLESGGGMVQTGGSLRLSCAASGFTFSDYAMSWVRQAPGKGLEWVSAISYDNG NTYYADSVKGRFTISRDNSKNALYLQMNSLRAEDTAVYYCAKVNHRFDYWGQGTLV TVSS | 144 |
| P015038 | EVQLQESGGGLVLPGGSLRLSCAASGFTFNNYAMSWVRQAPGKGLEWVSGISPNGS SIYYADSVKGRFTISRDNSKNTLYLQMNSLRAEDTAVYYCAKAARLSPSRNYSYAY GMDVWGQGTLVTVSS | 145 |
| P015039 | EVQLLESGGGLVQPGGSLRLSCAASGFTFSDYAMSWVRQAPGKGLEWVSWISPGGG NTYYADSVKGRFTISRDNSKNTLYLQMNSLRAEDTAVYYCAKGALMCLSATCSSDY GMDVWGQGTLVTVSS | 146 |
| P015040 | EVQLLESGGGLVQPGGSLRLSCAASGFTFSNYAMSWVRQAPGKGLEWVSGISPNGS SIYYADSVKGRFTISRDNSKNTLYLQMNSLRAEDTAVYYCAKAARLSPSRNYSYAY GMDVWGQGTLVTVSS | 147 |
| P015041 | EVQLLESGGGLVQPGGSLRLSCAASGFTFSNYAMSWVRQAPGKGLEWVSGISPNGS SIYYADSVKGRFTISRDNSKNTLYLQMNSLRAEDTAVYYCAKAARLSPSRNYSYAY GMDVWGQGTLVTVSS | 148 |
| P015042 | EVQLLESGGGLVQPGGSLRLSCAASGFTFSDYAMSWVRQAPGKGLEWVSVISSDGG SIYYADSVKGRFTISRDNSKNTLYLQMNSLRAEDTAVYYCAKDRSRDMEVDFDYWG QGTLVTVSS | 149 |

(continued)

| | | VH Sequence | SEQ ID NO: |
|---|---|---|---|
| | P015042v1 | EVQLLESGGGLVQPGGSLRLSCAASGFTFSDYAMSWVRQAPGKGLEWVSVISSDGG SIYYADSVKGRFTISRDNSKNTLYLQMNSLRAEDTAVYYCAKDRSRDMEVDFDYWG QGTLVTVSS | 150 |
| | P015043 | EVQLLESGGGLVQPGGSLRLSCAASGFTFSNYDMSWVRQAPGKGLEWVSWISPGGS SIYYADSVKGRFTISRDNSKNTLYLQMNSLRAEDTAVYYCAKTLTRFDYWGQGTLV TVSS | 151 |
| | P015044 | EVQLLESGGGLVQPGGSLRLSCAASGFTFSNYAMHWVRQAPGKGLEWVSIIYPDDD TRYNPSFRGRFTISRDNSKNTLYLQMNSLRAEDTAVYYCARDLLYAGDYWGQGTLV TVSS | 152 |
| | | VL Sequence | SEQ ID NO: |
| | P015001 | QSVLTQPPSASGTPGQRVTISCSGSSSNIGSNDVTWYQQLPGTAPKLLIYDDSKRP SGVPDRFSGSKSGTSASLAISGLRSEDEADYYCAAWDDSLSGYVFGGGTKLTVL | 153 |
| | P015002 | QSVLTQPPSASGTPGQRVTISCTGSSSNIGSNYVSWYRQLPGTAPKLLIYSDSNRP SGVPDRFSGSKSGTSASLAISGLRSEDEADYYCATWDASLSAYVFGGGTKLTVL | 154 |
| | P015003 | QSVLTQPPSASGTPGQRVTISCTGSSSNIGSNYVSWYRQLPGTAPKLLIYSDSNRP SGVPDRFSGSKSGTSASLAISGLRSEDEADYYCATWDASLSAYVFGGGTKLTVL | 155 |
| | P015004 | EIVLTQSPGTLSLSPGERATLSCRASQGISNYLAWYQQKPGQAPRLLIYDASNLES GIPDRFSGSGSGTDFTLTISRLEPEDFAVYYCQQYANLPYTFGQGTKVEIK | 156 |
| | P015005 | QSVLTQPPSASGTPGQRVTISCSGSSSNIGSNYVNWYQQLPGTAPKLLIYSDSHRP SGVPDRFSGSKSGTSASLAISGLRSEDEADYYCGSWDSSLNAYVLGGGTKLTVL | 157 |
| | P015006 | QSVLTQPPSASGTPGQRVTISCTGSSSNIGSNYVSWYRQLPGTAPKLLIYSDSNRP SGVPDRFSGSKSGTSASLAISGLRSEDEADYYCATWDASLSAYVFGGGTKLTVL | 158 |
| | P015007 | QSVLTQPPSASGTPGQRVTISCSGSSSNIGNNAVNWYQQLPGTAPKLLIYADSHRP SGVPDRFSGSKSGTSASLAISGLRSEDEADYYCGTWDSSLNGYVFGGGTKLTVL | 159 |
| | P015009 | QSVLTQPPSASGTPGQRVTISCTGSSSNIGSNYVSWYRQLPGTAPKLLIYSDSNRP SGVPDRFSGSKSGTSASLAISGLRSEDEADYYCGTWDSSLSGYVFGGGTKLTVL | 160 |
| | P015010 | QSVLTQPPSASGTPGQRVTISCTGSSSNIGNNNVTWYQQLPGTAPKLLIYSDSQRP SGVPDRFSGSKSGTSASLAISGLRSEDEADYYCGTWDYSLNGYVFGGGTKLTVL | 161 |
| | P015011 | QSVLTQPPSASGTPGQRVTISCTGSSSNIGNNYVSWYQQLPGTAPKLLIYSDSHRP SGVPDRFSGSKSGTSASLAISGLRSEDEADYYCGTWDYSLNGYVFGGGTKLTVL | 162 |

(continued)

| | VL Sequence | SEQ ID NO: |
|---|---|---|
| P015012 | QSVLTQPPSASGTPGQRVTISCSGSSSNIGNNAVNWYQQLPGTAPKLLIYANSHRP SGVPDRFSGSKSGTSASLAISGLRSEDEADYYCGTWDSSLSGYVFGGGTKLTVL | 163 |
| P015013 | QSVLTQPPSASGTPGQRVTISCSGSSSNIGSNSVYWYQQLPGTAPKPLIYSDSHRP SGVPDRFSGSKSGTSASLAISGLRSEDEADYYCGTWDYSLNAYVFGGGTKLTVL | 164 |
| P015014 | QSVLTQPPSASGTPGERVTISCSSSSSNIGSNYVNWYQQLPGTAPKLLIYDNSQRP SGVPDRFSGSKSGTSASLAISGLRSEDEADYYCGSWDDSLSGYVFGGGTKLTVL | 165 |
| P015015 | QSVLTQPPSASGTPGQRVTISCTGSSSNIGNNYVTWYQQLPGTAPRLLIYSDSQRP SGVPDRFSGSKSGTSASLAISGLRSEDEADYYCGTWDDSLNGYVFGGGTKLTVL | 166 |
| P015016 | QSVLTQPPSASGTPGQRVTISCSGSSSNIGSNTFNWYQQLPGTAPKLLIYADSHRP SGVPDRFSGSKSGTSASLAISGLRSEDEADYYCAAWDASLSAYVFGGGTKLTVL | 167 |
| P015017 | QSVLTQPPSASGTPGQRVTISCSGSSSNIGSNYVSWYQQLPGTAPKLLIYANSHRP SGVPDRFSGSKSGTSASLAISGLRSEDEADYYCGAWDSSLNGYVFGGGTKLTVL | 168 |
| P015018 | EIVLTQSPGTLSLSPGERATLSCRASQSVSTYLAWYQQKPGQAPRLLIYDASSLES GIPDRFSGSGSGTDFTLTISRLEPEDFAVYYCQQSYNLPHTFGQGTKVEIK | 169 |
| P015020 | QSVLTQPPSASGTPGQRVTISCTGSSSNIGNNTVNWYQQLPGTAPKLLIYADSNRP SGVPDRFSGSKSGTSASLAISGLRSEDEADYYCDTWDSSLNAYVFGGGTKLTVL | 170 |
| P015021 | QSVLTQPPSASGTPGQRVTISCSGSSSNIGNNSVTWYQQLPGTAPKLLIYYDSHRP SGVPDRFSGSKSGTSASLAISGLRSEDEADYYCGAWDSSLNGYVFGGGTKLTVL | 171 |
| P015022 | QSVLTQPPSASGTPGQRVTISCTGSSSNIGSNAVNWYQQLPGTAPKLLIYYDNQRP SGVPDRFSGSKSGTSASLAISGLRSEDEADYYCAAWDDSLSGYVFGGGTKLTVL | 172 |
| P015023 | QSVLTQPPSASGTPGQRVTLSCTGSSSNIGSNTVYWYQQLPGTAPKLLIYADSQRP SGVPDRFSGSKSGTSASLAISGLRSEDEADYYCGSWDSSLNAYVFGGGTKLTVL | 173 |
| P015024 | QSVLTQPPSASGTPGQRVTISCSGSSSNIGSNTFNWYQQLPGTAPKLLIYADSHRP SGVPDRFSGSKSGTSASLAISGLRSEDEADYYCATWDYSLSAYVFGGGTKLTVL | 174 |
| P015028 | QSVLTQPPSASGTPGQRVTISCSGSSSNIGSNTFNWYQQLPGTAPKLLIYANSHRP SGVPDRFSGSKSGTSASLAISGLRSEDEADYYCASWDASLSGYVFGGGTKLTVL | 175 |
| P015038 | QSVLTQPPSASGTPGQRVTISCTGSSSNIGNNNVTWYQQLPGTAPKLLIYSDSQRP SGVPDRFSGSKSGTSASLAISGLRSEDEADYYCGTWDYSLNGYVFGGGTKLTVL | 176 |
| P015039 | QSVLTQPPSASGTPGQRVTISCTGSSSNIGNNAVSWYQQLPGTAPKLLIYSDNQRP SGVPDRFSGSKSGTSASLAISGLRSEDEADYYCGTWDYSLNGYVFGGGTKLTVL | 177 |
| P015040 | QSVLTQPPSASGTPGQRVTISCTGSSSNIGNNNVTWYQQLPGTAPKLLIYSDSQRP SGVPDRFSGSKSGTSASLAISGLRSKDEADYYCGTWDYSLNGYVFGGGTKLTVL | 178 |

30

(continued)

| | VL Sequence | SEQ ID NO: |
|---|---|---|
| P015041 | QSVLTQPPSASGTPGQRVTISCTGSSSNIGNNNVTWYQQLPGTAPKLLIYSDSQRP SGVPDRFSGSKSGTSASLAISGLRSEDEADYYCGTWEYSLNGYVFGGGTKLTVL | 179 |
| P015042 | QSVLTQPPSASGTPGQRVTISCSGSSSNIGSNYVTWYQQLPGTAPKLLIYDSSQRP SGVPDRFSGSKSGTSASLAISGLRSEDEADYYCGTWDYSLSGYVFGGGTKLTVL | 180 |
| P015042v1 | QSVLTQPPSASGTPGQRVTISCSGSSSNIGSNYVTWYQQLPGTAPKLLIYDSSQRP SGVPDRFSGSKSGTSASLAISGLRSEDEADYYCGTWDYSLNGYVFGGGTKLTVL | 181 |
| P015043 | QSVLTQPPSASGTPGQRVTISCTGSSSNIGNNNVSWYQQLPGTAPKLLIYYDSHRP SGVPDRFSGSKSGTSASLAISGLRSEDEADYYCGTWDSSLSGYVFGGGTKLTVL | 182 |
| P015044 | QSVLTQPPSASGTPGQRVTISCGGNNIESKAVHWYQQLPGTAPKLLIYANNKRPSG VPDRFSGSKSGTSASLAISGLRSEDEADYYCQSYDSNKVVFGGGTKLTVL | 183 |

## Example 4: Analysis of binding domain of anti-ROR1 specific antibody (scFv)

ELISA was performed to analyze domains using scFvs of the 30 selected antibody clones described above. An expression vector capable of expressing the domain with the antigen for binding domain analysis was constructed followed by protein expression and purification. For production, residues corresponding to amino acids 1 to 542 or 165 to 395 of the ROR1 amino acid sequence indicated by UniProt ID: Q01973 were used. A gene block encoding the extracellular domain of ROR1 was constructed. The 3' end of the gene was conjugated with a His tag. A vector was obtained by introducing the gene into a pcDNA3.3 vector. Proteins were expressed and purified using transient transfection. Proteins were purified from the cell culture supernatant after culture at 8% $CO_2$, 37°C, and 130 rpm for 5 days. The culture fluid was passed through a column (GE Healthcare, 17-5438-01, MabSelect™ SuRe) to allow the expressed antibody to bind to the column. After elution with IgG elution buffer (Thermo Scientific, 21028, Pierce™ IgG Elution Buffer, pH 2.0), the eluted antibody fraction was concentrated by exchanging the buffer with PBS (pH 7.4) using an Amicon Ultra 30kDa tube (Merck Millipore, UFC903024, Amicon® Ultra-15 Centrifugal Filter Unit). The purified domain was quantified using absorbance and extinction coefficient at a wavelength of 280 nm.

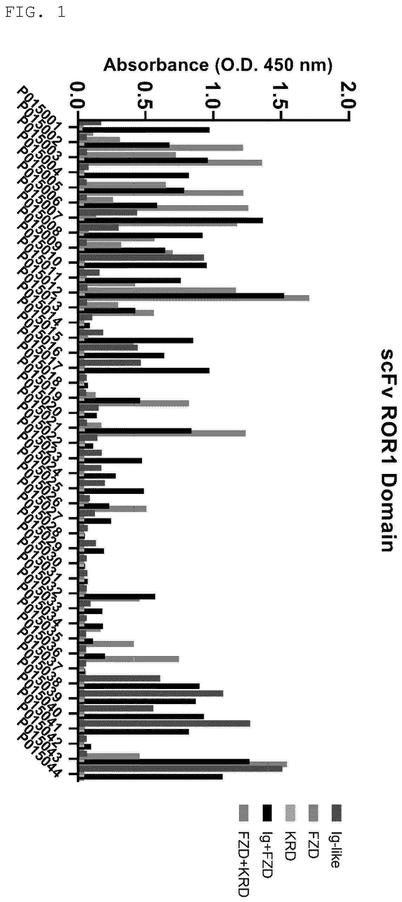

A 96-well plate was coated at 4°C for 16 hours with each of human ROR1-immunoglobulin domain (ACROBiosystems, RO1-H5221, Human/Cynomolgus/Rhesus macaque ROR1 (39-151, Ig-like domain) Protein, His Tag), human ROR1-frizzled domain (ACROBiosystems, RO1-H5222, Human/Cynomolgus/Rhesus macaque ROR1 (165-305, Frizzled domain) Protein, His Tag), human ROR1-kringle domain (ACROBiosystems, RO1-H5223, Human/-Cynomolgus/Rhesus macaque ROR1 (308-395, Kringle domain) Protein, His Tag), human ROR1-immunoglobulin-frizzled domain, and human ROR1-frizzled-kringle domain at a concentration of 2 µg/ml and then blocked using 3% skim milk. Thereafter, the plate was treated with each scFv and reacted for 2 hours, followed by washing four times using PBST and distilled water. After binding at room temperature for 1 hour using an HRP-conjugated anti-HA antibody (Roche, 12013819001, Anti-HA-Peroxidase, High Affinity) capable of binding to the HA tag, washing was performed six times using PBST and distilled water. After color development by adding TMB substrate solution (Thermo Scientific, 34029, 1-Step™ Ultra TMB-ELISA Substrate Solution), the color reaction was stopped with stop solution (Invitrogen, SS04, ELISA Stop Solution), and the absorbance was measured at OD 450 nm. The scFv binding to each domain was analyzed through ELISA. The epitope domains and binding properties were analyzed differently for individual clones, as shown in FIG. 1.

## Example 5: Mammalian cell expression and purification of anti-ROR1 antibody

Cloning and production were performed to produce each clone obtained in Example in the form of a complete immunoglobulin (IgG) monoclonal antibody. To construct a heavy chain expression vector, DNA encoding the heavy chain including the heavy chain variable region and constant region was cloned into a pOptivec vector. In addition, to construct a vector expressing the light chain, DNA encoding the light chain including the light chain variable region and the light chain constant region was cloned into a pcDNA3.3 vector.

Proteins were expressed and purified using the light chain and heavy chain expression vectors through transient transfection. Expi293F suspension cells (Gibco, A14527, Expi293F™ Cells) growing in suspension in Expi293 expression medium (Gibco, A1435101, Expi293™ Expression Medium) were transfected with a mixture of plasmid and Opti-MEM I (Gibco, 31985070, Opti-MEM™ I Reduced Serum Medium) and ExpiFectamine293 (Gibco, 100014995, ExpiFectamine™293). Proteins were purified from the cell culture supernatant after culture at 8% $CO_2$, 37°C, and 130 rpm for 5 days. The culture fluid was passed through a column (GE Healthcare, 17-5438-01, MabSelect™ SuRe) to allow the expressed antibody to bind to the column. After elution with IgG elution buffer (Thermo Scientific, 21028, Pierce™ IgG Elution Buffer, pH 2.0), the eluted antibody fraction was concentrated by exchanging the buffer with PBS (pH 7.4) using an Amicon Ultra 30kDa tube (Merck Millipore, UFC903024, Amicon® Ultra-15 Centrifugal Filter Unit). The purified anti-ROR1 antibody was quantified using absorbance and extinction coefficient at a wavelength of 280 nm.

## Example 6: Engineering for improving biological and physical properties of anti-ROR1 antibody

Antibody optimization was performed to remove undesired PTM sites of the antibodies obtained in Example above. Optimization was performed based on the P015042v1 antibody sequence, which showed excellent efficacy in the *in vitro* experiment results. Among antibody sequences, sequences that are liable to undergo deamidation and isomerization under stressed conditions during and after production due to undesired PTMs were found: HC 55G, LC S51, and LC G95A. It is known in existing papers that substituting N or D among sequences liable to undergo deamidation and isomerization significantly impairs the affinity of the antibody (Patel, C. N., Bauer, S. P., Davies, J., Durbin, J. D., Shiyanova, T. L., Zhang, K., & Tang, J. X. (2016). N+1 engineering of an aspartate isomerization hotspot in the complementarity-determining region of a monoclonal antibody. Journal of Pharmaceutical Sciences, 105(2), 512-518. https://doi.org/10.1016/s0022-3549(15)00185-9). Therefore, optimization was carried out by substituting the sequence following N or D among sequences.

Antibody affinity was measured by individually substituting the sequences described above through rational design. First, HC G55 was substituted with V (P015042v1-1) and K (P015042v1-2). Also, LC S51 was substituted with A (P015042v1-3) and K (P015042v1-4), and LC G95 was substituted with A (P015042v1-5). Based on results of production of new variants (P015042v1-1 to P015042v1-5) and measurement of affinity thereof, the HC G55 sequence was judged to be best substituted with K, and LC S51 and LC G95 with K and A, respectively. Therefore, P015042v1-6 having all three sequence modifications was constructed.

The CDR and heavy chain and light chain variable region sequences of the modified antibodies are shown in Tables 7 and 8 below.

[Table 7]

| | CDR H1 | | CDR H2 | | CDR H3 | |
|---|---|---|---|---|---|---|
| | Sequence | SEQ ID NO: | Sequence | SEQ ID NO: | Sequence | SEQ ID NO: |
| P015042v1-1 | DYAMS | 10 | VISSDVGSIYYADSVKG | 184 | DRSRDMEVDFDY | 63 |
| P015042v1-2 | DYAMS | 10 | VISSDKGSIYYADSVKG | 185 | DRSRDMEVDFDY | 63 |
| P015042v1-3 | DYAMS | 10 | VISSDGGSIYYADSVKG | 39 | DRSRDMEVDFDY | 63 |
| P015042v1-4 | DYAMS | 10 | VISSDGGSIYYADSVKG | 39 | DRSRDMEVDFDY | 63 |
| P015042v1-5 | DYAMS | 10 | VISSDGGSIYYADSVKG | 39 | DRSRDMEVDFDY | 63 |
| P015042v1-6 | DYAMS | 10 | VISSDKGSIYYADSVKG | 185 | DRSRDMEVDFDY | 63 |
| | CDR L1 | | CDR L2 | | CDR L3 | |
| | Sequence | SEQ ID NO: | Sequence | SEQ ID NO: | Sequence | SEQ ID NO: |
| P015042v1-1 | SGSSSNIGSNYVT | 84 | DSSQRPS | 101 | GTWDYSLNGYV | 109 |
| P015042v1-2 | SGSSSNIGSNYVT | 84 | DSSQRPS | 101 | GTWDYSLNGYV | 109 |
| P015042v1-3 | SGSSSNIGSNYVT | 84 | DASQRPS | 186 | GTWDYSLNGYV | 109 |
| P015042v1-4 | SGSSSNIGSNYVT | 84 | DKSQRPS | 187 | GTWDYSLNGYV | 109 |
| P015042v1-5 | SGSSSNIGSNYVT | 84 | DSSQRPS | 101 | GTWDYSLNAYV | 110 |

(continued)

| | CDR L1 | | CDR L2 | | CDR L3 | |
|---|---|---|---|---|---|---|
| | Sequence | SEQ ID NO: | Sequence | SEQ ID NO: | Sequence | SEQ ID NO: |
| P015042v1-6 | SGSSSNIGSNYVT | 84 | DKSQRPS | 187 | GTWDYSLNAYV | 110 |

[Table 8]

| | VH Sequence | SEQ ID NO: |
|---|---|---|
| P015042v1-1 | EVQLLESGGGLVQPGGSLRLSCAASGFTFSDYAMSWVRQAPGKGLEWVSVISSDVGSIYYADSVKGRFTISRDNSKNTLYLQMNSLRAEDTAVYYCAKDRSRDMEVDFDYWGQGTLVTVSS | 188 |
| P015042v1-2 | EVQLLESGGGLVQPGGSLRLSCAASGFTFSDYAMSWVRQAPGKGLEWVSVISSDKGSIYYADSVKGRFTISRDNSKNTLYLQMNSLRAEDTAVYYCAKDRSRDMEVDFDYWGQGTLVTVSS | 189 |
| P015042v1-3 | EVQLLESGGGLVQPGGSLRLSCAASGFTFSDYAMSWVRQAPGKGLEWVSVISSDGGSIYYADSVKGRFTISRDNSKNTLYLQMNSLRAEDTAVYYCAKDRSRDMEVDFDYWGQGTLVTVSS | 190 |
| P015042v1-4 | EVQLLESGGGLVQPGGSLRLSCAASGFTFSDYAMSWVRQAPGKGLEWVSVISSDGGSIYYADSVKGRFTISRDNSKNTLYLQMNSLRAEDTAVYYCAKDRSRDMEVDFDYWGQGTLVTVSS | 191 |
| P015042v1-5 | EVQLLESGGGLVQPGGSLRLSCAASGFTFSDYAMSWVRQAPGKGLEWVSVISSDGGSIYYADSVKGRFTISRDNSKNTLYLQMNSLRAEDTAVYYCAKDRSRDMEVDFDYWGQGTLVTVSS | 192 |
| P015042v1-6 | EVQLLESGGGLVQPGGSLRLSCAASGFTFSDYAMSWVRQAPGKGLEWVSVISSDKGSIYYADSVKGRFTISRDNSKNTLYLQMNSLRAEDTAVYYCAKDRSRDMEVDFDYWGQGTLVTVSS | 193 |
| | **VL Sequence** | SEQ ID NO: |
| P015042v1-1 | QSVLTQPPSASGTPGQRVTISCSGSSSNIGSNYVTWYQQLPGTAPKLLIYDSSQRPSGVPDRFSGSKSGTSASLAISGLRSEDEADYYCGTWDYSLNGYVFGGGTKLTVL | 194 |
| P015042v1-2 | QSVLTQPPSASGTPGQRVTISCSGSSSNIGSNYVTWYQQLPGTAPKLLIYDSSQRPSGVPDRFSGSKSGTSASLAISGLRSEDEADYYCGTWDYSLNGYVFGGGTKLTVL | 195 |
| P015042v1-3 | QSVLTQPPSASGTPGQRVTISCSGSSSNIGSNYVTWYQQLPGTAPKLLIYDASQRPSGVPDRFSGSKSGTSASLAISGLRSEDEADYYCGTWDYSLNGYVFGGGTKLTVL | 196 |

(continued)

| | VL Sequence | SEQ ID NO: |
|---|---|---|
| P015042v1-4 | QSVLTQPPSASGTPGQRVTISCSGSSSNIGSNYVTWYQQLPGTAPKLLIYDKSQR<br><br>PSGVPDRFSGSKSGTSASLAISGLRSEDEADYYCGTWDYSLNGYVFGGGTKLTVL | 197 |
| P015042v1-5 | QSVLTQPPSASGTPGQRVTISCSGSSSNIGSNYVTWYQQLPGTAPKLLIYDSSQR<br><br>PSGVPDRFSGSKSGTSASLAISGLRSEDEADYYCGTWDYSLNAYVFGGGTKLTVL | 198 |
| P015042v1-6 | QSVLTQPPSASGTPGQRVTISCSGSSSNIGSNYVTWYQQLPGTAPKLLIYDKSQR<br><br>PSGVPDRFSGSKSGTSASLAISGLRSEDEADYYCGTWDYSLNAYVFGGGTKLTVL | 199 |

## Example 7: Analysis of specific binding ability of anti-ROR1 antibody to ROR1 (ELISA)

ELISA was performed to analyze specific binding ability to an antigen using the IgG antibody of each clone selected in Example above.

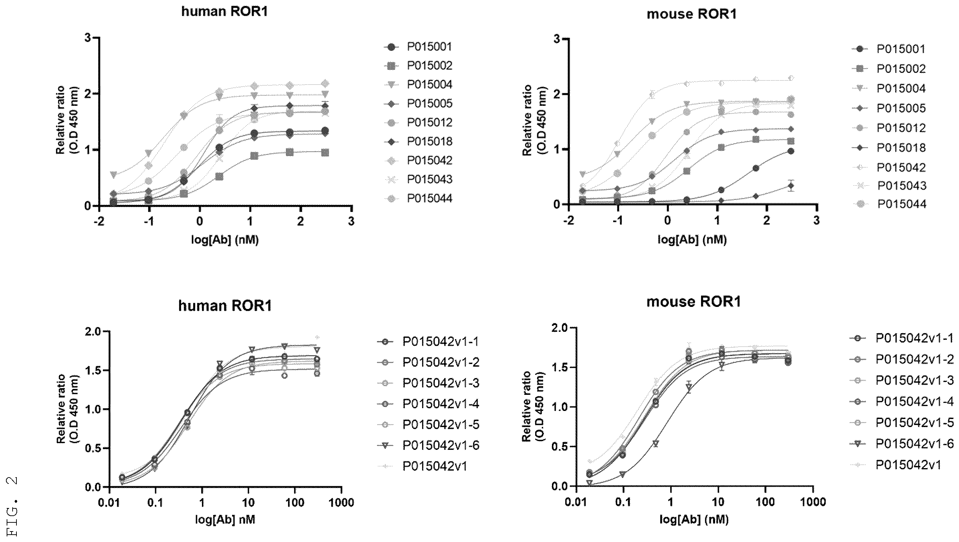

A 96-well plate was coated at 4°C for 16 hours with each of human ROR1 and mouse ROR1 proteins at a concentration of 2 $\mu$g/ml and then blocked using 3% skim milk. Thereafter, the plate was treated with each antibody at concentrations of 300 nM, 60 nM, 12 nM, 2.4 nM, 0.48 nM, 0.096 nM, and 0.0192 nM and reacted for 1 hour. After washing with PBST and distilled water, binding at room temperature for 1 hour using a goat-derived HRP-conjugated anti-human antibody (Invitrogen, 31482, Goat anti-Human IgG F(ab')$_2$ Secondary Antibody, HRP) and then washing again using PBST and distilled water were performed. After color development by adding TMB substrate solution (Thermo Scientific, 34029, 1-Step™ Ultra TMB-ELISA Substrate Solution), the color reaction was stopped with stop solution (Invitrogen, SS04, ELISA Stop Solution), and the absorbance was measured at OD 450 nm. It was confirmed that the anti-ROR1 antibody of the present invention and the modified antibody thereof with improved biological and physical properties cross-linked to human ROR1 and mouse ROR1 (FIG. 2).

## Example 8: Analysis of specific binding ability of anti-ROR1 antibody to ROR1 (SPR)

To more quantitatively analyze the binding ability of the anti-ROR1 antibody to ROR1, SPR (surface plasmon resonance) was performed. A Biacore 3000 device (GE Healthcare) was used.

Using the amine coupling method, human ROR1 or mouse ROR1 was diluted with 10 mM sodium acetate, pH 4.5 and immobilized on a CM5 sensor chip (GE Healthcare) at 300 response units (RU). The activated portion remaining on the surface of the sensor chip was deactivated by adding 1 M ethanolamine-HCl (pH 8.5). The anti-ROR1 antibody of the present invention was injected at concentrations of 300, 150, 75, 37.5, 18.75, 9.375, and 4.6875 nM for 180 seconds into the antibody protein immobilized on the CM5 sensor chip (Ka) followed by separation at the same flow rate for additional 180 seconds (Kd), thereby analyzing KD binding sensorgrams. Thereby, as shown in FIG. 2, the anti-ROR1 antibody exhibited sensorgrams specifically binding to human ROR1 and mouse ROR1, and the final KD values through Ka and Kd values (1:1 Langmuir 1:1 kinetics) are shown in Tables 9 and 10 below.

[Table 9]

| Antibody | Species | Ka [1/Ms] | Kd [1/s] | KD [M] |
|---|---|---|---|---|
| P015001 | Human/ Monkey | 4.18e6 | 2.64e-3 | 6.30e-10 |
| P015002 | | 5.43e4 | 2.02e-4 | 3.71e-9 |
| P015004 | | 1.06e7 | 3.43e-3 | 3.25e-10 |
| P015005 | | 1.69e5 | 6.78e-5 | 4.00e-10 |
| P015012 | | 4.07e6 | 3.21e-4 | 7.89e-11 |
| P015018 | | 2.95e6 | 2.49e-3 | 8.45e-10 |
| P015042 | | 1.41e6 | 2.39e-4 | 1.7e-10 |
| P015042v1 | | 1.62e6 | 2.78e-4 | 1.72e-10 |

(continued)

| Antibody | Species | Ka [1/Ms] | Kd [1/s] | KD [M] |
|---|---|---|---|---|
| P015042v1-1 | | 2.19e6 | 7.22e-5 | 3.30e-11 |
| P015042v1-2 | | 2.66e6 | 4.08e-5 | 1.52e-11 |
| P015042v1-3 | | 3.03e6 | 7.95e-5 | 2.62e-11 |
| P015042v1-4 | | 2.04e6 | 4.34e-6 | 2.13e-11 |
| P015042v1-5 | | 2.53e6 | 1.89e-5 | 7.48e-12 |
| P015042v1-6 | | 1.72e6 | 1.57e-4 | 9.12e-11 |
| P015043 | | 5.58e5 | 1.6e-3 | 2.87e-9 |
| P015044 | | 1.24e6 | 3.82e-3 | 3.08e-9 |

[Table 10]

| Antibody | Species | Ka [1/Ms] | Kd [1/s] | KD [M] |
|---|---|---|---|---|
| P015001 | Mouse | 5.64e5 | 0.129 | 2.30e-7 |
| P015002 | | 4.64e4 | 2.78e-4 | 6.00e-9 |
| P015004 | | 8.18e6 | 2.69e-3 | 3.28e-10 |
| P015005 | | 1.20e5 | 1.00e-4 | 8.33e-10 |
| P015012 | | 1.25e6 | 3.56e-4 | 2.85e-10 |
| P015018 | | 9.40e5 | 0.0782 | 8.32e-8 |
| P015042 | | 1.72e6 | 1.76e-4 | 1.02e-10 |
| P015042v1 | | 1.94e6 | 3.40e-4 | 1.75e-10 |
| P015042v1-1 | | 2.24e6 | 4.30e-4 | 1.92e-10 |
| P015042v1-2 | | 2.71e6 | 2.26e-4 | 8.31e-11 |
| P015042v1-3 | | 4.30e6 | 5.48e-4 | 1.27e-10 |
| P015042v1-4 | | 2.14e6 | 2.82e-4 | 1.32e-10 |
| P015042v1-5 | | 2.54e6 | 1.06e-5 | 4.16e-11 |
| P015042v1-6 | | 1.87e6 | 1.50e-3 | 8.02e-10 |
| P015043 | | 6.46e | 1.4e-3 | 2.17e-9 |
| P015044 | | 1.67e6 | 2.02e-3 | 1.21e-9 |

**Example 9: ROR1 domain mapping of anti-ROR1 antibody**

ELISA was performed to analyze the binding domain using the IgG antibody of the selected clone.

A 96-well plate was coated at 4°C for 16 hours with each of human ROR1-immunoglobulin domain (ACROBiosystems, RO1-H5221, Human/Cynomolgus/Rhesus macaque ROR1 (39-151, Ig-like domain) Protein, His Tag), human ROR1-frizzled domain (ACROBiosystems, RO1-H5222, Human/Cynomolgus/Rhesus macaque ROR1 (165-305, Frizzled domain) Protein, His Tag), human ROR1-kringle domain (ACROBiosystems, RO1-H5223, Human/-Cynomolgus/Rhesus macaque ROR1 (308-395, Kringle domain) Protein, His Tag), human ROR1-immunoglobulin-frizzled domain, and human ROR1-frizzled-kringle domain at a concentration of 2 $\mu$g/ml and then blocked using 3% skim milk. Thereafter, the plate was treated with each antibody at concentrations of 300 nM, 60 nM, 12 nM, 2.4 nM, 0.48 nM, 0.096 nM, and 0.0192 nM and reacted for 1 hour. After washing with PBST and distilled water, binding at room temperature for 1 hour using a goat-derived HRP-conjugated anti-human antibody (Invitrogen, 31482, Goat anti-Human IgG F(ab')$_2$ Secondary Antibody, HRP) and then washing again using PBST and distilled water were performed. After color development by adding TMB substrate solution (Thermo Scientific, 34029, 1-Step™ Ultra TMB-ELISA Substrate Solution), the color reaction was stopped with stop solution (Invitrogen, SS04, ELISA Stop Solution), and the absorbance was measured at OD 450 nm. The anti-ROR1 antibody clone binding to each domain was analyzed through ELISA (FIG.

3).

**Example 10: Quantitative analysis of ROR1 receptor expression by cell type**

Each of T-47D, Jeko-1 (abnormal cell line), AMB-BT-0024T, AMB-BT-0016T, AMB-BT-0013T, AMB-LC-0002T, AMB-LC-0003T, and KUC-OC21-025T (abnormal patient-derived cells) was dispensed in duplicate at $2 \times 10^5$ cells in a 96-well plate. 100 nM of each of mouse IgG1-PE isotype (Invitrogen, 12-4714-82) and ROR1-2A2-PE (BioLegend, 357804) was dispended per well. The cells were incubated at 4°C for 30 minutes, during which Quantibrite PE beads (BD, 340495) were added to 0.5 ml of PBS, followed by flow cytometry using a BD FACSAria flow cytometer. A reference point for ROR1 expression was set using beads and the number of expressed ROR1 receptors depending on the cell type was quantified by analyzing the ROR1 expression level in the cells treated with mouse isotype and ROR1-2A2-PE antibody compared to beads.

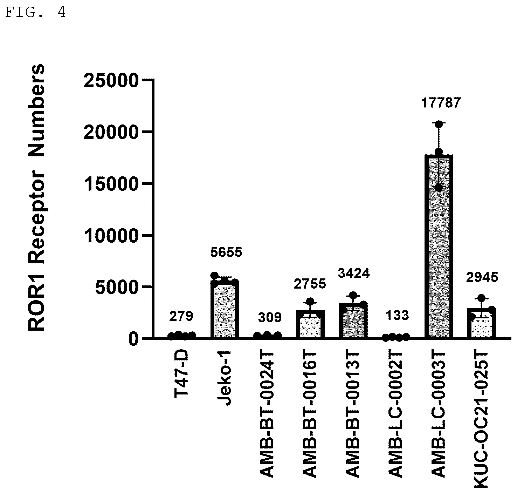

Jeko-1, a human lymphoma cell line, showed high ROR1 expression, and among patient-derived cells (PDXC), AMB-LC-0003T, patient-derived lung cancer cells, showed the highest ROR1 expression (FIG. 4).

[Table 11]

| Cell | Cell type | Cancer type | ROR1 receptor count (qFAC) |
|---|---|---|---|
| T-47D | Cell line | Breast cancer | 279 |
| Jeko-1 | Cell line | Mantle cell lymphoma | 5,655 |
| AMB-BT-0024T | PDXC | Glioblastoma | 309 |
| AMB-BT-0016T | PDXC | Glioblastoma | 2,755 |
| AMB-BT-0013T | PDXC | Glioblastoma | 3,424 |
| AMB-LC-0002T | PDXC | Lung cancer | 133 |
| AMB-LC-0003T | PDXC | Lung cancer | 17,787 |
| KUC-OC21-025T | PDC | Ovarian cancer | 2,945 |

**Example 11: Measurement of specific binding ability of anti-ROR1 antibody to ROR1 expressed on cell surface**

Before therapeutic use of anti-ROR1 antibodies, it is very important to determine whether the antibody binds to the antigen expressed on the cell surface. The cell binding ability of anti-ROR1 antibodies developed by the present inventors was measured using FACS in Jeko-1 cell line and AMB-LC-0003T patient-derived cells, which were confirmed to have high ROR1 expression. Each cell type was dispensed at $1 \times 10^5$ cells/100 $\mu$l FACS buffer per well in a 96-well plate.

Cell binding ability analysis was performed using anti-ROR1 candidate antibodies P015004, P015042, P015043, P015044, P015042v1 with improved biological and physical properties, and P015042v1-6, one of modified materials thereof. Each antibody was serially diluted 10-fold from 100 nM to 10 nM, 1 nM, 100 pM, 10 pM, and 1 pM and the cells were treated therewith. After reaction at 4°C for 30 minutes, washing was performed twice. PE-labeled goat anti-human IgG Fc PE (ThermoFisher; 12-4998-82) as a secondary antibody was diluted at a ratio of 1:100 in FACS buffer, and 100 $\mu$l thereof was dispensed per well. Reaction again at 4°C for 30 minutes and washing twice were performed. Finally, 100 $\mu$l of FACS buffer was dispensed per well, followed by mixing well with a pipette and then flow cytometry using a NovoCyte flow cytometer (Agilent). Analysis was performed using GraphPad Prism 9.3.1, and $EC_{50}$ values were obtained using a log(agonist) vs. response - Variable slope nonlinear graph model.

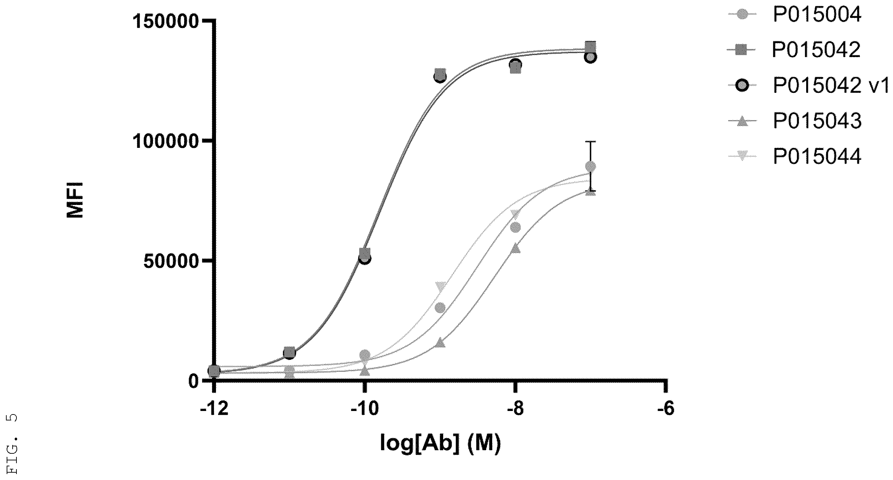

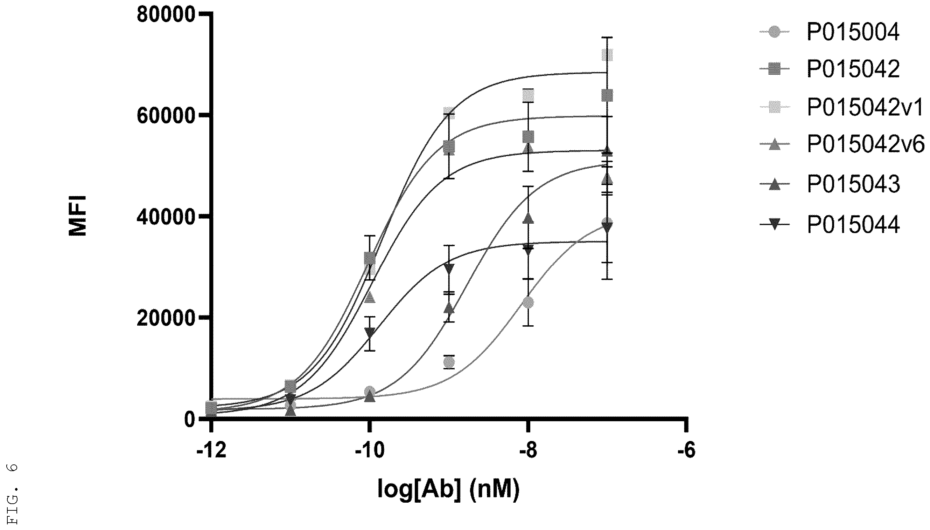

Based on the results of cell binding ability, AMB-LC-0003T patient-derived cells, which had the highest ROR1 expression, exhibited higher MFI (mean fluorescence intensity) than the Jeko-1 cell line. The antibodies that showed subnanomolar $EC_{50}$ in both cell types were P015042, and P015042v1 and P015042v1-6 obtained by additionally modifying the sequences thereof, and P015044 also showed nanomolar $EC_{50}$. The other anti-ROR1 clones, such as P015004 and P015043, also showed $EC_{50}$ of <10 nM, and thus all showed good cell binding ability (FIGs. 5 and 6).

[Table 12]

| Results of analysis of binding ability to ROR1 antigen expressed in AMB-LC-0003T patient-derived lung cancer cells | |
|---|---|
| **Hits** | **$EC_{50}$ (nM)** |
| P015004 | 4.60 |
| P015042 | 0.15 |
| P015042v1 | 0.16 |
| P015043 | 5.30 |
| P015044 | 1.78 |

[Table 13]

| Results of analysis of binding ability of antibodies to ROR1 antigen expressed in Jeko-1 cell line | |
|---|---|
| **Hits** | **$EC_{50}$ (nM)** |
| P015004 | 8.16 |
| P015042 | 0.081 |
| P015042v1 | 0.014 |
| P015042v1-6 | 0.011 |
| P015043 | 1.70 |
| P015044 | 0.13 |

**Example 12: Analysis of cellular internalization of anti-ROR1 antibody**

In order to develop an antibody-drug conjugate, the anti-ROR1 antibody has not only to bind to the ROR1 antigen in the cell but also to be internalized into the cell so that the drug of the conjugate may penetrate into the cell to cause reaction. To analyze this, internalization was confirmed by detecting the ROR1 antigen remaining on the cell surface at various time points. Jeko-1 cells were dispensed in duplicate at $1 \times 10^5$ cells/100 μl per well, and plates were prepared for respective time points: at 0 minutes, 10 minutes, 30 minutes, 1 hour, 2 hours, and 4 hours after the start of cellular internalization, and ROR1 receptors that were not internalized by the antibody but remained on the cell surface were confirmed. The cells were treated with 1 nM of each antibody and then reacted at 4°C for 30 minutes. After centrifugation at 1,100 g for 2 minutes, washing was performed twice. In the remaining plates except for the plate at 0 minutes, the cells were mixed with 100 μl of FACS buffer and reacted at 37°C and 5% $CO_2$. In the plate at 0 minutes, goat anti-human IgG Fc PE (ThermoFisher, 12-4998-82) was diluted at a ratio of 1:100, dispensed in an amount of 100 μl per well, and reacted at 4°C for about 30 minutes. After washing twice, flow cytometry was performed using a NovoCyte flow cytometer (Agilent), and the other plates were treated in the same manner at individual times to confirm ROR1 expression remaining in Jeko-1 cells. Values were standardized based on the ROR1 expression level confirmed at 0 minutes, and analyzed using an [Inhibitor] vs. normalized response - Variable slope nonlinear graph model in GraphPad Prism 9.3.1.

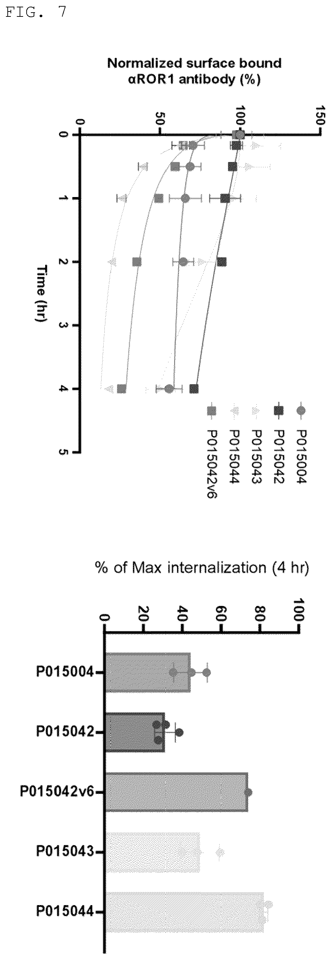

Cellular internalization varied depending on each antibody clone, and most antibodies were confirmed to be continuously internalized even after 4 hours. P015042v1-6, which was engineered to improve biological and physical properties of the P015042 antibody, exhibited a similar level to Merck's UC-961 antibody, which had the best cellular internalization (FIG. 7).

**Example 13: Production of anti-ROR1 antibody-drug conjugate**

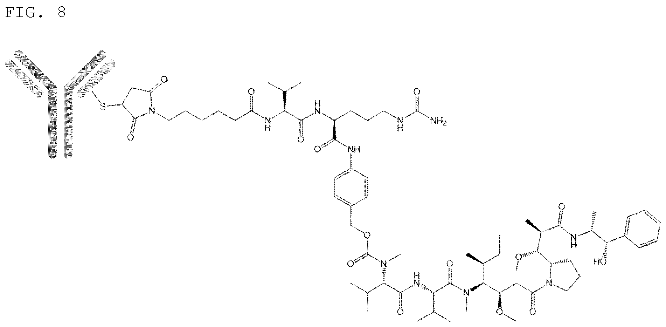

A general method of producing an antibody-drug conjugate according to the present invention or an intermediate thereof is described. The names and abbreviation symbols of the compounds included in each reaction formula are shown and described (FIG. 8).

The antibody-drug conjugate represented by formula (1), in which an antibody (Y) and a drug-linker (LP) structure are connected via thioether, may be produced, for example, by the following method.

[Formula (1)]          X (1) + LP (2) → X-LP (3)

The drug-linker (LP) used is maleimidocaproyl-valine-citrulline-p-aminobenzyloxycabonyl monomethyl auristatin E (vc-PAB-MMAE;

CCC(C)C(C(CC(=O)N1CCCC1C(C(C)C(=O)NC(C)C(C2=CC=CC=C2)O)OC)OC)N (C)C(=O)C(C(C)C)NC(=O)C(C(C)C)N(C)C(=O)OCC3=CC=C(C=C3)NC(=O)C(    CCCNC(=O)N)NC(=O)C(C(C)C)NC(=O)CCCCCN4C(=O)C=CC4=O), and X is an IgG1 monoclonal antibody having a sulfhydryl group.

An antibody-drug conjugate may be produced through reaction between the maleimidyl group of LP and the sulfhydryl group of X.

The antibody (X) having a sulfhydryl group may be obtained according to the method described below. The antibody may be reacted with a reducing agent such as tris(2-carboxyethyl)phosphine hydrochloride (TCEP) to thus reduce the disulfide bond in the hinge region of the antibody, thereby forming a sulfhydryl group.

Specifically, 2 to 20 molar equivalents of 5 mM TCEP was used as a reducing agent per disulfide in the antibody, and reacted with an antibody (Y) in buffer containing 5 mM ethylenediaminetetraacetic acid (EDTA), a representative chelating agent, thereby producing an antibody (X) having a sulfhydryl group in which disulfide in the antibody was partially or fully reduced. The disulfide reduction reaction in the antibody described above was carried out at 37°C for 2 hours. The buffer used is the same phosphate buffered saline (pH 7.4) used in antibody production. The disulfide reduction reaction in the antibody described above was carried out at 37°C for 2 hours.

In order to remove impurities such as TCEP and EDTA remaining after completion of reaction, the antibody (X) solution having a sulfhydryl group was added to an Amicon Ultra (30 kDa, Millipore Co.) and then centrifuged (at 3950G for 10 to 20 minutes) using a centrifuge (Eppendorf centrifuge 5810 R), and the buffer was exchanged with phosphate buffered saline (pH 7.4). This process was repeated a total of three times.

The antibody (X) having a sulfhydryl group from which impurities were removed through buffer exchange was recovered from the Amicon Ultra (30 kDa, Millipore Co.) and reacted with 5 to 15 molar equivalents of 10 mM drug-linker (LP) at room temperature for 2 hours. Since the drug-linker (LP) may be modified by light, this reaction was carried out in the dark. To increase the solubility of the compound, the drug-linker (LP) was dissolved in dimethylacetamide (DMA), a representative organic solvent. About 10 to 20% v/v of DMA was added to buffer containing the antibody (X) having a sulfhydryl group.

The antibody-drug conjugation reaction may be terminated by inactivating reactivity of the unreacted drug-linker (LP) with a thiol-containing reagent. The thiol-containing reagent used is N-acetyl-L-cysteine (NAC). Specifically, reaction was terminated by adding 5 molar equivalents of NAC to the antibody-drug conjugation reaction solution and storing the same at room temperature for 30 minutes.

In order to remove the residual drug-linker and NAC, the antibody-drug reaction solution was added to the Amicon Ultra (30 kDa, Millipore Co.) and then centrifuged (at 3950G for 10 to 20 minutes) using a centrifuge (Eppendorf centrifuge 5810 R), and the buffer was exchanged with phosphate buffered saline (pH 7.4). This process was repeated a total of three times. Finally, the antibody-drug conjugate was recovered from the filtration membrane in the Amicon Ultra (30 kDa, Millipore Co.).

**Example 14: Analysis of concentration of anti-ROR1 antibody-drug conjugate**

The antibody concentration in the antibody-drug conjugate was measured using a spectrophotometer (Thermo-Fisher Scientific NanoDrop 8000).

**Example 15: Analysis of purity of anti-ROR1 antibody-drug conjugate**

The purity of the antibody-drug conjugate was measured by size exclusion high-performance liquid chromatography (SEC-HPLC) analysis employing the method described below. As pretreatment for analysis, the antibody-drug conjugate sample is prepared at a concentration of 0.5 mg/ml and a total volume of 25 μl. SEC-HPLC analysis is performed under the following conditions.

[Table 14]

| SEC-HPLC system configuration | |
| --- | --- |
| HPLC system | Alliance e2695 (Waters Corporation) |
| Column | TSKgel G3000SWXL (0008541, Tosoh Bioscience) |
| Column and sample temperature | 25°C |
| Amount of sample injected | 25 μl |
| Mobile phase | Phosphate buffered saline + 0.15 M sodium chloride + 0.01% v/v sodium azide |

(continued)

| SEC-HPLC system configuration | |
| --- | --- |
| Detection wavelength | 280 nm |
| Flow rate | 0.5 ml/min |

The purity of the antibody-drug conjugate was measured by comparing and contrasting the retention time of each peak appearing in the antibody-drug conjugate chromatogram with the retention time for each peak of the size marker (Gel Filtration Standard, Bio-Rad, 1511901). In size exclusion chromatography, a larger molecule is detected first, and thus the peak that appears first based on the antibody peak (150 kDa) was analyzed to be a polymer aggregate generated due to hydrophobicity of the drug and the peak that appears subsequently was analyzed to be the drug that was conjugated and then separated. The purity of the antibody-drug conjugate itself was calculated by converting the area of the peak corresponding to 150 kDa in the entire chromatogram into a percentage.

**Example 16: Analysis of average number of conjugated drug molecules per antibody molecule of anti-ROR1 antibody-drug conjugate (DAR analysis)**

The average number of conjugated drug molecules per antibody molecule (drug-to-antibody ratio, DAR) of the antibody-drug conjugate was measured through hydrophobic interaction high-performance liquid chromatography (HIC-HPLC) employing the method described below. As pretreatment for HIC-HPLC analysis, the antibody-drug conjugate sample to be used for analysis is prepared at a concentration of 0.5 mg/ml and a total volume of 30 $\mu$l. HIC-HPLC analysis was performed under the following conditions.

[Table 15]

| HIC-HPLC system configuration | |
| --- | --- |
| HPLC system | Agilent 1290 1260 Infinity II Bio Prime HPLC System (Agilent Technologies Inc.) |
| Column | TSKgel butyl-NPR (0042168, Tosoh Bioscience) |
| Column and sample temperature | 25°C |
| Amount of sample injected | 25 $\mu$l |
| Mobile phase A | 25 mM potassium hydrogen phosphate + 1.5 M ammonium sulfate (pH 7.0) |
| Mobile phase B | 25 mM potassium hydrogen phosphate + isopropyl alcohol (20% v/v) (pH 7.2) |
| Detection wavelength | 280 nm |
| Flow rate | 0.5 ml/min |

[Table 16]

| HIC-HPLC gradient program | | |
| --- | --- | --- |
| Time (min) | % Mobile phase A | % Mobile phase B |
| 0 | 100 | 0 |
| 0.3 | 100 | 0 |
| 20 | 0 | 100 |
| 20.1 | 100 | 0 |
| 30 | 100 | 0 |

Compared to the same drug-unconjugated antibody, the antibody-drug conjugate exhibits higher hydrophobicity in proportion to the number of conjugated drug molecules and therefore has a longer retention time. A total of four disulfide bonds is present in the antibody, and two drugs may be conjugated per disulfide bond. Accordingly, up to five peaks (DAR 0, DAR 2, DAR 4, DAR 6, and DAR 8) may be observed in the HIC-HPLC chromatogram of the antibody-drug conjugate. The peaks that appear sequentially after the retention time of the drug-unconjugated antibody (DAR 0) were designated in the order of DAR 2, DAR 4, DAR 6, and DAR 8 described above. Once the DAR assignment to each peak was completed, the average number of conjugated drug molecules per antibody molecule was calculated using the following equation.

$$DAR = \sum_{n=0}^{8} \frac{Area\ of\ each\ peak \times n_{drug}}{Area\ of\ total\ peak};$$

$n_{drug}$ = Number of drug molecules assigned to corresponding peak

**Example 17: Analysis of binding ability of anti-ROR1 antibody-drug conjugate to antigen**

Affinity analysis of antibody-drug conjugates serves to evaluate equivalence to the parent antibody and also determine whether potential affinity is impaired from processes such as disulfide bond reduction and drug-linker conjugation in the production of an antibody having a sulfhydryl group. This analysis was conducted using an enzyme-linked immunosorbent assay (ELISA) described below.

A 96-well plate (Costar 3590, Corning) was coated by dispensing 50 $\mu$l of 2 $\mu$g/ml human- or mouse-derived ROR1 antigen (Sino Biological) per well. After shaking off the coated ROR1 antigen, the plate was blocked by dispensing 200 $\mu$l of 3% skim milk per coated well. After 1 hour, the 3% skim milk was shaken off, the parent antibody and the antibody-drug conjugate were serially diluted 1/5-fold seven times from the highest concentration of 300 nM, and 50 $\mu$l thereof was dispensed in the designated well. After primary antigen-antibody binding for 1 hour, the plate was washed with 0.05% v/v surfactant (PBST) and distilled water (three times each). Then, Fab-HRP was diluted in 3% skim milk at a ratio of 1:3000 and 50 $\mu$l thereof was dispensed per well. After secondary binding reaction for 1 hour, the plate was washed with 0.05% PBST and tertiary distilled water (three times each). After removing the residual water from the plate, 50 $\mu$l of TMB (34029, ThermoFisher) was dispensed per well and reacted for 3 to 5 minutes. Finally, reaction was terminated by adding 50 $\mu$l of stop solution (SS04, ThermoFisher) to each well.

[Table 17]

| Affinity analyzer and conditions | |
| --- | --- |
| Analyzer | Infinite M200 Pro (Tecan) |
| Analysis wavelength | 450 nm |
| Data processing | GraphPad Prism 9 |

When the binding ability of the antibody-drug conjugate to the antigen was 80% or more compared to a monoclonal antibody, the binding ability of the antibody-drug conjugate to the antigen was judged to be similar.

The three types of anti-ROR1 antibody-drug conjugates (P015004-vc-PAB-MMAE, P015042-vc-PAB-MMAE, and P015044-vc-PAB-MMAE) thus produced were evaluated according to the aforementioned antibody-drug conjugate analysis and QC method, and the results thereof are summarized in Table 18 below.

[Table 18]

| Anti-ROR1 antibody-drug conjugate analysis and QC | | | |
| --- | --- | --- | --- |
| Antibody-drug conjugate | Purity (%) | Average number of conjugated drug molecules per antibody molecule | Affinity compared to parent antibody (%) |
| P015004-vc-PAB-MMAE | 98.9 | 3.0 | 80.3 |
| P015042-vc-PAB-MMAE | 97.0 | 3.3 | 99.3 |
| P015044-vc-PAB-MMAE | 98.6 | 3.0 | 82.7 |

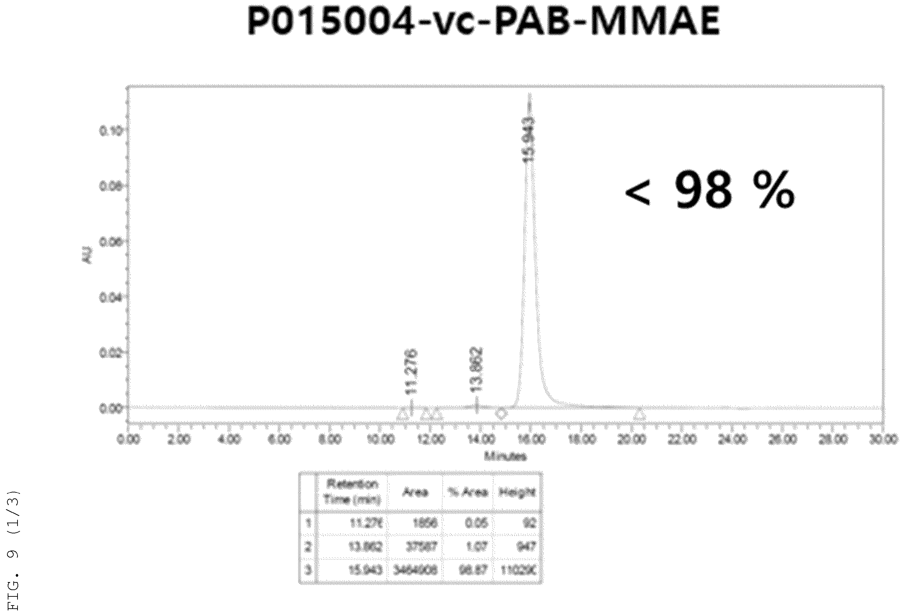

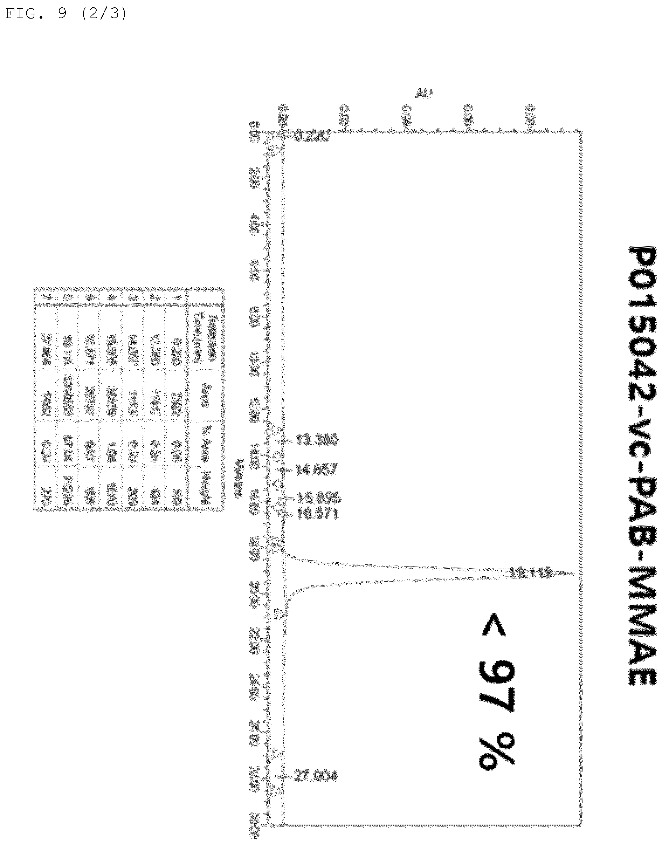

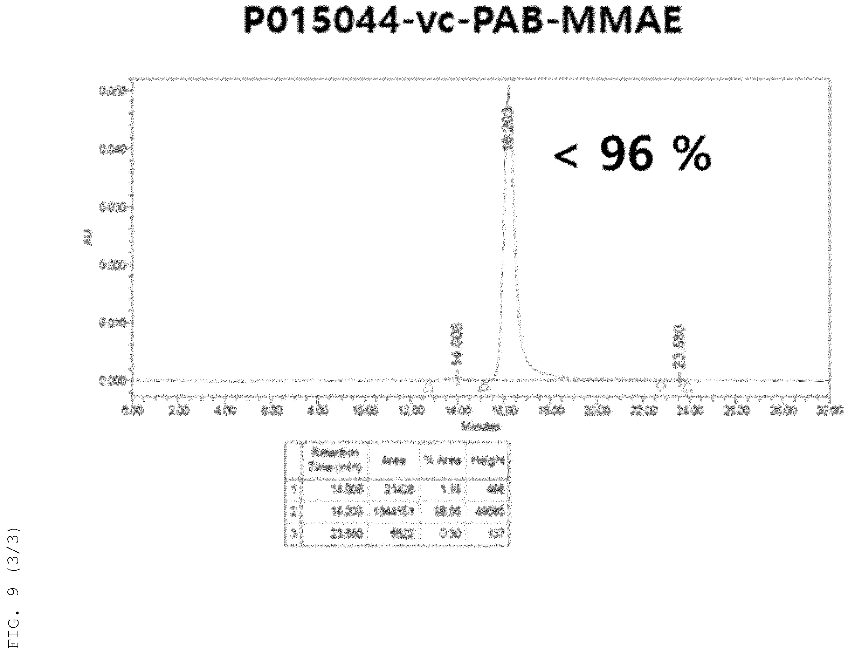

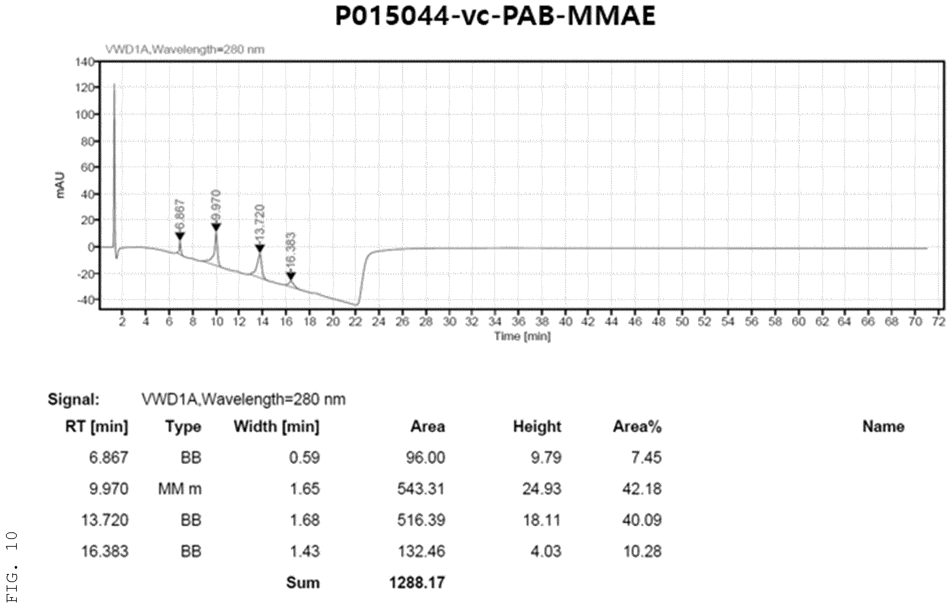

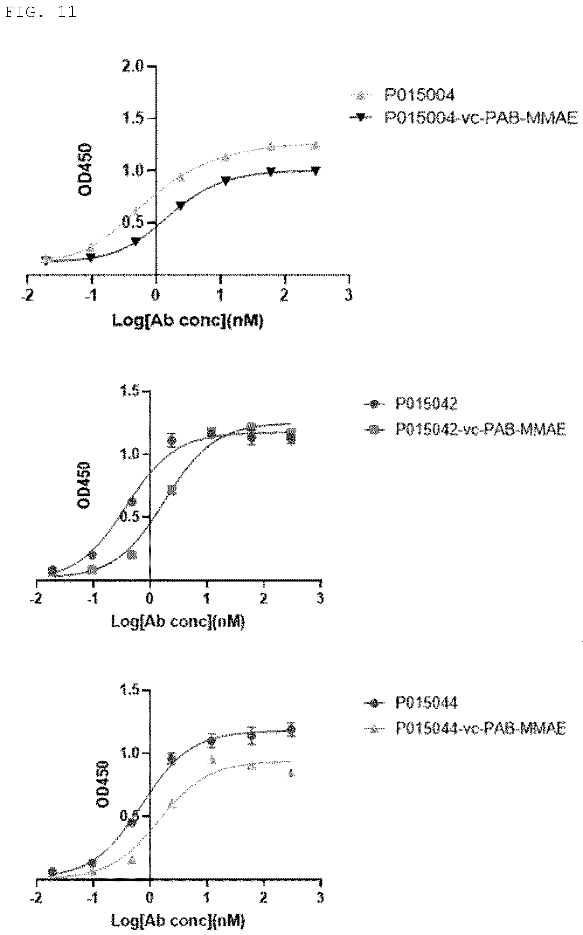

FIG. 9 shows results of purity analysis of the antibody-drug conjugates used in Example of the present invention, FIG. 10 shows results of analysis of the average number of conjugated drug molecules per antibody molecule of the antibody-drug conjugate used in Example of the present invention, and FIG. 11 shows results of ELISA for ROR1 binding of the antibody-drug conjugates used in Example of the present invention.

**Example 18: *In vitro* cytotoxicity evaluation of anti-ROR1 antibody-drug conjugate**

The toxicity of the anti-ROR1 candidate antibody-drug conjugates described above as an anticancer drug was evaluated in AMB-LC-0003T known as patient-derived lung cancer cells with high ROR1 expression and AMB-LC-0002T with very low ROR1 expression.

In each ultra-low attachment U bottom 384-well transparent plate (S-Bio, #MS-9384UZ), patient-derived lung cancer cells AMB-LC-0003T and AMB-LC-0002T cells were dispensed in triplicate at 500 cells/well in 40 $\mu$l of M10018 (Aimedbio) media. After centrifugation at 250 g for 2 minutes, reaction was carried out at 37°C and 5% $CO_2$ for 24 hours. Thereafter, each antibody-drug conjugate was serially diluted 3-fold from 500 nM to 0.314 pM and the cells were treated therewith. The plate was reacted again at 37°C and 5% $CO_2$ for 6 days.

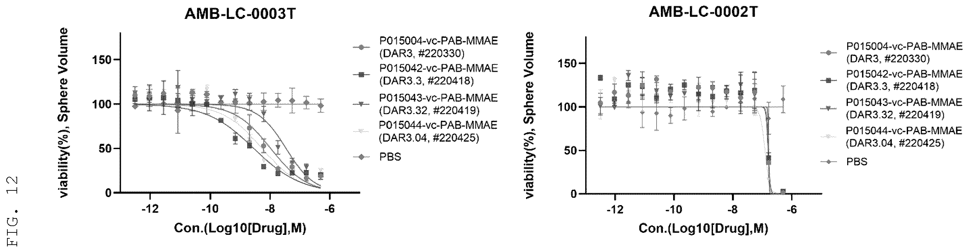

After a total of 7 days of reaction, spheroids were formed by the cells in the 384-well plate, so images of spheroids in each well were captured using Operetta CLS (PerkinElmer) according to a high-content screening method. To maintain the spheroid form of the cells in each well, washing was not performed before analysis. The captured images were converted into volume through additional analysis, and the spheroid volume values by concentration were standardized based on the spheroid treated with the lowest concentration of drug. Graphs were created with a log(inhibitor) vs. normalized response - Variable slope using GraphPad Prism 9.3.1, and $IC_{50}$ values were obtained (FIG. 12).

[Table 19]

|  |  | P015004-vc-PAB-MMAE (DAR3, #220330) | P015042-vc-PAB-MMAE (DAR3. 3, #220418) | P015043-vcPAB-MMAE (DAR3.32, #220419) | P015044-vc-PAB-MMAE (DAR3.04, #220425) |
|---|---|---|---|---|---|
| AMB-LC-0003T | IC50 (nM) | 10.62 | 2.88 | 34.56 | 5.38 |
| AMB-LC-0002T | IC50 (nM) | 159.5 | 162.1 | 165.2 | 122.4 |
| AMB-LC-0002T /AMB-LC-0003T | Fold Change | 15.0 | 56.3 | 4.8 | 22.7 |

[Table 20]

| Patient Derived Cell |  | P015042-vc-PAB-MMAE (DAR3.3, #220418) | P015044-vc-PAB-MMAE (DAR3.04, #220425) | UC961-vc-PAB-MMAE (DAR2.7, #220517) |
|---|---|---|---|---|
| AMB-LC-0003T | IC50 (nM) | 2.30 | 6.59 | 25.44 |
| AMB-LC-0002T | IC50 (nM) | 186.9 | 261.3 | 281.6 |
| AMB-LC-0002T /AMB-LC-0003T | Fold Change | 81.4 | 39.7 | 11.1 |

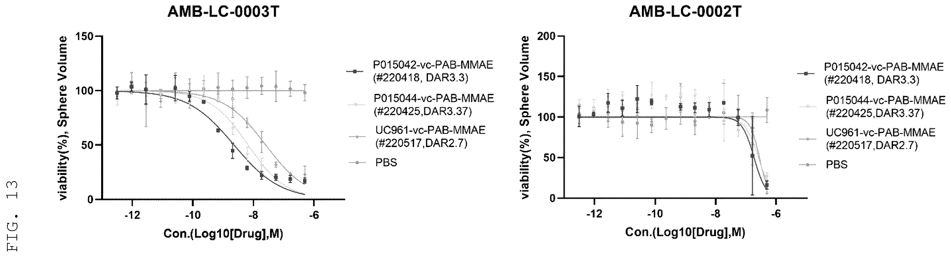

The P015042-vc-PAB-MMAE antibody-drug conjugate described above exhibited excellent $IC_{50}$ (2.30 nM) in AMB-LC-0003T with high ROR1 expression, and only showed a ROR1-specific effect with $IC_{50}$ of 100 nM or more in AMB-LC-0002T, patient-derived cells with low ROR1 expression. The relative potency thereof was about 8 times that of UC961-vc-PAB-MMAE, an antibody-drug conjugate disclosed in U.S. Patent No. 10,335,496 B2, enabling expectation of ROR1-specific anticancer efficacy as an antibody-drug conjugate (FIG. 13).

**INDUSTRIAL APPLICABILITY**

The anti-ROR1 antibody or antigen-binding fragment thereof according to the present invention, can exhibit superior binding ability to ROR1 compared to existing anti-ROR1 antibodies, and can be efficiently used for desired tumor or cancer prevention or treatment.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

**SEQUENCE LISTING FREE TEXT**

An electronic file is attached.

**Claims**

1. An antibody or antigen-binding fragment thereof that specifically binds to ROR1 (receptor tyrosine kinase like orphan receptor 1), comprising:

a heavy chain CDR1 comprising at least one amino acid sequence selected from the group consisting of SEQ ID NO: 1 to SEQ ID NO: 16,
a heavy chain CDR2 comprising at least one amino acid sequence selected from the group consisting of SEQ ID NO: 17 to SEQ ID NO: 41, SEQ ID NO: 184, and SEQ ID NO: 185,
a heavy chain CDR3 comprising at least one amino acid sequence selected from the group consisting of SEQ ID NO: 42 to SEQ ID NO: 65,
a light chain CDR1 comprising at least one amino acid sequence selected from the group consisting of SEQ ID NO: 66 to SEQ ID NO: 86,
a light chain CDR2 comprising at least one amino acid sequence selected from the group consisting of SEQ ID NO: 87 to SEQ ID NO: 102, SEQ ID NO: 186, and SEQ ID NO: 187, and
a light chain CDR3 comprising at least one amino acid sequence selected from the group consisting of SEQ ID NO: 103 to SEQ ID NO: 121.

2. The antibody or antigen-binding fragment thereof according to claim 1, in which comprising a heavy chain variable region comprising at least one amino acid sequence selected from the group consisting of SEQ ID NO: 122 to SEQ ID NO: 152 and SEQ ID NO: 188 to SEQ ID NO: 193.

3. The antibody or antigen-binding fragment thereof according to claim 1, in which comprising a light chain variable region comprising at least one amino acid sequence selected from the group consisting of SEQ ID NO: 153 to SEQ ID NO: 183 and SEQ ID NO: 194 to SEQ ID NO: 199.

4. The antibody or antigen-binding fragment thereof according to claim 1, in which comprising a single-chain Fv (scFv), single-chain antibody, Fab, F(ab'), and disulfide-linked Fv (sdFv).

5. The antibody or antigen-binding fragment thereof according to claim 4, wherein the scFv comprises a heavy chain variable region comprising at least one amino acid sequence selected from the group consisting of SEQ ID NO: 122 to SEQ ID NO: 152 and SEQ ID NO: 188 to SEQ ID NO: 193 and a light chain variable region comprising at least one amino acid sequence selected from the group consisting of SEQ ID NO: 153 to SEQ ID NO: 183 and SEQ ID NO: 194 to SEQ ID NO: 199 are connected via a linker.

6. The antibody or antigen-binding fragment thereof according to claim 5, wherein the linker comprises $(G_nS)_m$ (in which each of n and m is 1 to 10).

7. A nucleic acid encoding the antibody or antigen-binding fragment thereof according to any one of claims 1 to 6.

8. A recombinant expression vector comprising the nucleic acid according to claim 7.

9. A host cell transfected with the recombinant expression vector according to claim 8.

10. The host cell according to claim 9, wherein the host cell is COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, or HT1080.

11. A method of producing an antibody or antigen-binding fragment thereof that specifically binds to ROR1, comprising:

producing an antibody by culturing the host cell according to claim 9; and
isolating and purifying the produced antibody.

12. A bispecific or multispecific antibody comprising the antibody or antigen-binding fragment thereof according to any one of claims 1 to 6.

13. An immune cell-engaging bispecific or multispecific antibody comprising an scFv of the antibody according to any one of claims 1 to 6 and an scFv comprised of a second binding domain comprising at least one scFv of an antibody that binds to an immune cell-activating antigen.

14. An antibody-drug conjugate (ADC) in which the antibody or antigen-binding fragment thereof according to any one of claims 1 to 6 is conjugated to a drug.

15. The antibody-drug conjugate according to claim 14, wherein the drug is at least one selected from the group consisting of maytansinoid, auristatin (including MMAE and MMAF), aminopterin, actinomycin, bleomycin, talisomycin, camptothecin, N8-acetylspermidine, 1-(2-chloroethyl)-1,2-dimethyl sulfonyl hydrazide, esperamicin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-$\alpha$, interferon-$\gamma$, tumor necrosis factor, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins derived from bacteria or animals and plants.

16. The antibody-drug conjugate according to claim 14, wherein the antibody or antigen-binding fragment thereof is conjugated to the drug via a linker.

17. The antibody-drug conjugate according to claim 16, wherein the linker is a cleavable linker or a non-cleavable linker.

18. The antibody-drug conjugate according to claim 17, wherein the cleavable linker is an acid-labile linker, a disulfide linker, a peptide linker, or a beta-glucuronide linker, or the non-cleavable linker comprises a thioether group or a maleimidocaproyl group.

19. The antibody-drug conjugate according to claim 16, wherein the linker is conjugated to a cysteine residue exposed during reduction of a disulfide bond of the antibody or to a cysteine residue present in a tag attached to the antibody.

20. A chimeric antigen receptor (CAR) comprising an extracellular domain comprising an antigen-binding site, a transmembrane domain, and an intracellular signaling domain, wherein the antigen-binding site of the extracellular domain is an scFv of the antibody according to any one of claims 1 to 6.

21. An immune cell into which the chimeric antigen receptor (CAR) according to claim 20 is introduced.

22. The immune cell according to claim 21, wherein the immune cell is at least one selected from the group consisting of T cells, NK cells, cytokine induced killer cells (CIKs), activated cytotoxic T lymphocytes (CTLs), macrophages, tumor-infiltrating lymphocytes (TILs), and dendritic cells.

23. A composition for preventing or treating cancer, comprising the antibody or antigen-binding fragment thereof according to any one of claims 1 to 6, a bispecific or multispecific antibody comprising the antibody or antigen-binding fragment thereof, an antibody-drug conjugate comprising the antibody or antigen-binding fragment thereof, a chimeric antigen receptor comprising the antibody or antigen-binding fragment thereof, or an immune cell comprising the chimeric antigen receptor.

24. A method of preventing or treating cancer, comprising administering the antibody or antigen-binding fragment thereof according to any one of claims 1 to 6, a bispecific or multispecific antibody comprising the antibody or antigen-binding fragment thereof, an antibody-drug conjugate comprising the antibody or antigen-binding fragment thereof, a chimeric antigen receptor comprising the antibody or antigen-binding fragment thereof, or an immune cell comprising the chimeric antigen receptor.

FIG. 1

FIG. 2

FIG. 3

### Ig-like domain

### Frizzled domain

### Kringle domain

### IF domain

### FK domain

FIG. 4

FIG. 5

FIG. 6

FIG. 7

FIG. 8

FIG. 9 (1/3)

# P015042-vc-PAB-MMAE

**< 97 %**

| | Retention Time (min) | Area | % Area | Height |
|---|---|---|---|---|
| 1 | 0.220 | 2822 | 0.08 | 169 |
| 2 | 13.380 | 11910 | 0.35 | 424 |
| 3 | 14.657 | 11103 | 0.33 | 209 |
| 4 | 15.895 | 35659 | 1.04 | 1070 |
| 5 | 16.571 | 29787 | 0.87 | 806 |
| 6 | 19.119 | 3316558 | 97.04 | 91225 |
| 7 | 27.904 | 9962 | 0.29 | 270 |

FIG. 9 (2/3)

FIG. 9 (3/3)

FIG. 10

PO15044-VC-PAB-MMAE

Signal: VWD1A,Wavelength=280 nm

| RT [min] | Type | Width [min] | Area | Height | Area% | Name |
|----------|------|-------------|--------|--------|-------|------|
| 6.867 | BB | 0.59 | 96.00 | 9.79 | 7.45 | |
| 9.970 | MM m | 1.65 | 543.31 | 24.93 | 42.18 | |
| 13.720 | BB | 1.68 | 516.39 | 18.11 | 40.09 | |
| 16.383 | BB | 1.43 | 132.46 | 4.03 | 10.28 | |
| | | Sum | 1288.17 | | | |

FIG. 11

FIG. 12

**AMB-LC-0003T**

**AMB-LC-0002T**

FIG. 13

**AMB-LC-0003T**

**AMB-LC-0002T**

## INTERNATIONAL SEARCH REPORT

| International application No. |
|---|
| **PCT/KR2023/010847** |

| | |
|---|---|
| **A.** | **CLASSIFICATION OF SUBJECT MATTER** |
| | **C07K 16/28**(2006.01)i; **A61K 47/68**(2017.01)i; **A61P 35/00**(2006.01)i; **A61K 39/00**(2006.01)i |

According to International Patent Classification (IPC) or to both national classification and IPC

| | |
|---|---|
| **B.** | **FIELDS SEARCHED** |

Minimum documentation searched (classification system followed by classification symbols)

C07K 16/28(2006.01); A61K 39/00(2006.01); A61K 47/68(2017.01); A61P 35/00(2006.01); C07K 16/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: ROR1(receptor tyrosine kinase like orphan receptor 1), 항체(antibody), scFv, 중쇄(heavy chain), 경쇄(light chain), 핵산(nucleic acid), 발현벡터(expression vector), 형질감염(transfection), 숙주세포 (host cell), 항체-약물 접합체(antibody-drug conjugate, ADC), 키메라 항원 수용체(chimeric antigen receptor, CAR), 암 (cancer), 예방(prevention), 치료(treatment)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| A | KR 10-2018-0069067 A (NBE-THERAPEUTICS AG) 22 June 2018 (2018-06-22)<br>   See abstract, claims 1-4, and paragraphs [0020]-[0045]. | 1-23 |
| A | KR 10-2018-0101554 A (THE SCRIPPS RESEARCH INSTITUTE et al.) 12 September 2018 (2018-09-12)<br>   See entire document. | 1-23 |
| A | WO 2017-136607 A1 (FRED HUTCHINSON CANCER RESEARCH CENTER) 10 August 2017 (2017-08-10)<br>   See entire document. | 1-23 |
| A | WO 2021-159029 A1 (VELOSBIO INC.) 12 August 2021 (2021-08-12)<br>   See entire document. | 1-23 |

| | |
|---|---|
| ✔ Further documents are listed in the continuation of Box C. | ✔ See patent family annex. |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 October 2023** | **31 October 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2023/010847** |

**C.    DOCUMENTS CONSIDERED TO BE RELEVANT**

| Category* | Citation of document, with indication, where appropriate, of the relevant passages | Relevant to claim No. |
|---|---|---|
| A | WO 2022-129622 A1 (ALMAC DISCOVERY LIMITED) 23 June 2022 (2022-06-23)<br>See entire document. | 1-23 |
| A | KR 10-2020-0143470 A (EXELIXIS, INC.) 23 December 2020 (2020-12-23)<br>See entire document. | 1-23 |

Form PCT/ISA/210 (second sheet) (July 2022)

**INTERNATIONAL SEARCH REPORT**

| International application No. |
|---|
| **PCT/KR2023/010847** |

| Box No. I    Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

    ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/KR2023/010847**

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **24**
   because they relate to subject matter not required to be searched by this Authority, namely:

   Claim 24 pertains to a method for treatment of the human body by surgery or therapy, as well as a diagnostic method (PCT Article 17(2)(a)(i) and PCT Rule 39.1(iv)).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2023/010847**

| Patent document cited in search report | | | Publication date (day/month/year) | Patent family member(s) | | | Publication date (day/month/year) |
|---|---|---|---|---|---|---|---|
| KR | 10-2018-0069067 | A | 22 June 2018 | CN | 108884160 | A | 23 November 2018 |
| | | | | CN | 108884160 | B | 03 February 2023 |
| | | | | EP | 3368574 | A1 | 05 September 2018 |
| | | | | JP | 2019-502363 | A | 31 January 2019 |
| | | | | US | 2019-0153092 | A1 | 23 May 2019 |
| | | | | WO | 2017-072361 | A1 | 04 May 2017 |
| KR | 10-2018-0101554 | A | 12 September 2018 | CN | 108848669 | A | 20 November 2018 |
| | | | | CN | 108848669 | B | 07 June 2022 |
| | | | | EP | 3405496 | A1 | 28 November 2018 |
| | | | | JP | 2019-509021 | A | 04 April 2019 |
| | | | | JP | 7000660 | B2 | 04 February 2022 |
| | | | | US | 10618959 | B2 | 14 April 2020 |
| | | | | US | 11242388 | B2 | 08 February 2022 |
| | | | | US | 2018-0340026 | A1 | 29 November 2018 |
| | | | | US | 2020-0299381 | A1 | 24 September 2020 |
| | | | | US | 2022-0153839 | A1 | 19 May 2022 |
| | | | | WO | 2017-127664 | A1 | 27 July 2017 |
| WO | 2017-136607 | A1 | 10 August 2017 | CN | 108699149 | A | 23 October 2018 |
| | | | | CN | 108699149 | B | 06 January 2023 |
| | | | | EP | 3411408 | A1 | 12 December 2018 |
| | | | | EP | 3411408 | B1 | 08 December 2021 |
| | | | | EP | 4006056 | A1 | 01 June 2022 |
| | | | | JP | 2019-511904 | A | 09 May 2019 |
| | | | | JP | 2022-028918 | A | 16 February 2022 |
| | | | | JP | 7029401 | B2 | 03 March 2022 |
| | | | | US | 10968275 | B2 | 06 April 2021 |
| | | | | US | 2019-0040132 | A1 | 07 February 2019 |
| | | | | US | 2021-0147540 | A1 | 20 May 2021 |
| WO | 2021-159029 | A1 | 12 August 2021 | CN | 115052896 | A | 13 September 2022 |
| | | | | EP | 4100438 | A1 | 14 December 2022 |
| | | | | JP | 2023-512774 | A | 29 March 2023 |
| | | | | KR | 10-2022-0139916 | A | 17 October 2022 |
| | | | | US | 2023-0021388 | A1 | 26 January 2023 |
| WO | 2022-129622 | A1 | 23 June 2022 | KR | 10-2023-0121130 | A | 17 August 2023 |
| KR | 10-2020-0143470 | A | 23 December 2020 | CN | 112384533 | A | 19 February 2021 |
| | | | | EP | 3797122 | A1 | 31 March 2021 |
| | | | | JP | 2021-522162 | A | 30 August 2021 |
| | | | | US | 2021-0155692 | A1 | 27 May 2021 |
| | | | | WO | 2019-204564 | A1 | 24 October 2019 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016172726 A1
- WO 2001077342 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- WO 2011117330 A
- US 20170368169
- WO 2004010957 A
- WO 2015182984 A
- US 6319494 B
- US 7741465 B
- US 7446190 B
- US 9212229 B
- US 8822647 B
- US 10316102 B
- US 10336810 B
- US 10335496 B2

### Non-patent literature cited in the description

- **KLEIN et al.** *J. Exp. Med*, 2001, vol. 194, 1625
- **CHOI et al.** *Cell Stem Cell*, 2018, vol. 22, 951-959
- **BAI, XUELIAN** ; **KIM, JIHYE** ; **KANG, SEUNGMIN** ; **KIM, WANKYU** ; **SHIM, HYUNBO**. A Novel Human scFv Library with Non-Combinatorial Synthetic CDR Diversity. *PloS one*, 2015, vol. 10, e0141045
- **YANG, HYE** ; **KANG, KYUNG** ; **TCW, JULIA** ; **HIM, HYUNBO.** Construction of a large synthetic human scFv library with six diversified CDRs and high functional diversity. *Molecules and cells.*, 2009, vol. 27, 225-35
- **PATEL, C. N.** ; **BAUER, S. P.** ; **DAVIES, J** ; **DURBIN, J. D** ; **SHIYANOVA, T. L.** ; **ZHANG, K., &AMP** ; **TANG, J. X**. N+1 engineering of an aspartate isomerization hotspot in the complementarity-determining region of a monoclonal antibody. *Journal of Pharmaceutical Sciences*, 2016, vol. 105 (2), 512-518, https://doi.org/10.1016/s0022-3549(15)00185-9